# EUROPEAN PATENT APPLICATION

(11) **EP 3 557 583 A1**
(43) Date of publication of application: **23.10.2019**
(21) Application number: 17883175.6
(22) Date of filing: 06.12.2017
(51) Int. Cl.: G16H 10/60

(54) **ASSISTANCE CONTROL METHOD AND ASSISTANCE SYSTEM**

(30) Priority: 19.12.2016 JP 2016245203
(71) Applicant: Konica Minolta, Inc., Tokyo 100-7015 (JP)
(72) Inventor: NISHIKADO, Masashi, Tokyo 100-7015 (JP); NODA, Atsuhiro, Tokyo 100-7015 (JP); OKADA, Hiroshi, Tokyo 100-7015 (JP); KITAMURA, Ken, Tokyo 100-7015 (JP)
(74) Representative: Alton, Andrew
(86) International application number: PCT/JP2017/043793
(87) International publication number: WO 2018/116830

(57) **Abstract**

An assistance control method for an assistance system that assists monitoring a monitoring object person includes: an acquisition step of acquiring, from among a plurality of terminal devices, a notification destination terminal device which is a terminal device of a notification destination to be notified of an abnormality of the monitoring object person and is determined in accordance with an abnormal state of the monitoring object person determined by a central processing device; an abnormality notification step of notifying, of the abnormality of the monitoring object person, the notification destination terminal device acquired in the acquisition step; and an information transmission step of transmitting monitoring object person information to the notification destination terminal device from the central processing device in accordance with a transmission request for the monitoring object person information from the notification destination terminal device that has been notified of abnormality of the monitoring object person in the abnormality notification step.

## Description

### Technical Field

The present invention relates to an assistance control method and an assistance system which assist monitoring a monitoring object person who is a monitoring target.

### Background Art

Our country (Japan) comes to have an aging society, more specifically, a super-aging society in which an aging rate of a population aged 65 and older to the total population exceeds 21% due to improvement in living standards, improvement in sanitary conditions, and improvement in medical standards along with the rapid economic growth after the war. According to the statistics of September 2013, the elderly population is approximately 31.86 million people, the population aging rate thereof is 25.0%, and one out of four people is an elderly person. Additionally, it is estimated in that the elderly population will be approximately 37.41 million people, and one out of three people will be an elderly person in year 2035 (Statistics Bureau of the Ministry of Internal Affairs and Communications of Japan). In such an aging society, it is presumed that the number of persons requiring nursing and care (persons requiring nursing care) due to illness, injury, aging, and the like will be increased more than that in the number of the persons requiring nursing care in a normal society that is not the aging society. Moreover, our country is also a declining-birth rate society in which the total fertility rate of year 2013 is 1.43, for example. Therefore, there is a problem of "care of an elderly person by another elderly person" in which an elderly person requiring nursing care is cared by another elderly person in a family (such as a spouse, child, or sibling).

The elderly persons requiring nursing care enter hospitals and facilities such as welfare facilities for aged people (short-stay facilities, elderly nursing homes, special elderly nursing home, and the like referred by Japanese statutory laws), and receive nursing and care. In these facilities, there may be situations in which, for example, a person requiring nursing care is injured by falling from a bed, collapsing during walking, or the like, or sneaks out of a bed for loitering. In the event of such situations, immediate attendance is necessary. Additionally, if such situations are left without taking any countermeasure, a more serious situation may be caused. For this reason, in the above-described facilities, nurses and care workers (nursing care givers) confirm safety and a state thereof through periodic patrol. Then, the nurses, care workers, and the like record results thereof as nursing records and care records (nursing care records).

However, the nursing industry and the care industry are facing a problem of chronic labor shortage due to a slower increase in the number of nursing care givers than increase in the number of the persons requiring nursing care. Furthermore, a workload per person is heavier during hours in semi-night duty and night duty than during hours in day duty because of decrease in the number of nursing care givers, and therefore, reduction of the workload is required. Moreover, the above-described problem of "care of an elderly person by another elderly person" is no exception in the above-mentioned facilities, and there often may be a case where an elderly person requiring nursing care is cared by an elderly nursing care giver. Generally, since physical strength of a person declines with age even though the one is healthy, a workload is heavier compared to a workload of a young carer, and one's motion and judgment speed becomes slow.

To solve the problem of labor shortage and reduce the workloads of the nursing care givers, a technology to assist nursing work and care work is demanded. For this reason, in recent years, research and development have been made on an assistance technology to assist monitoring a monitoring object person who is a monitoring target to be monitored (i.e., a person requiring nursing care).

As such a technology, an abnormality notification device has been conventionally proposed (see Patent Literature 1, for example). In the abnormality notification device disclosed in Patent Literature 1, when an abnormality of a patient is detected, reliability of a detection result of the abnormality is calculated by using data erroneously detected when the abnormality is erroneously detected in the past, and a notification destination to be notified of the abnormality of the patient is changed between a case where the calculated reliability is high and a case where the calculated reliability is low. Patent Literature 1 discloses an example in which in a case where the reliability of the abnormality detection result is high, all of medical staff members concerned with patient's medical care are notified, and in the case where the reliability is low, only a nurse concerned with the patient's medical care is notified (see the paragraph [0058] of Patent Literature 1).

### Citation List

### Patent Literature

Patent Literature 1: JP 2016-130886 A

### Summary of Invention

According to above Patent Literature 1, as described above, in a case where reliability of an abnormality detection result is high, all of medical staff members concerned with patient's medical care are notified, and in a case where the reliability is low, only a nurse concerned with the patient's medical care is notified. However, it is not constantly necessary to notify all of the medical staff members just because the reliability of the abnormality detection result of the patient is high. Also, it is not constantly enough to notify the nurse just because the reliability of the abnormality detection result of the patient is low. Thus, the notification destination of the patient's abnormality is not adequately studied in Patent Literature 1.

The present invention is made in view of the above circumstances and is directed to providing an assistance control method and an assistance system in which an appropriate notification destination is notified of an abnormality of a monitoring object person when the monitoring object person is determined abnormal.

To achieve the above-described object, an aspect of the present invention is an assistance control method for an assistance system that assists monitoring a monitoring object person and includes:
a sensor device which is provided in a manner corresponding to the monitoring object person who is a monitoring target, and detects monitoring object person information that is information associated with the monitoring object person;
a central processing device which is communicably connected to the sensor device, receives, from the sensor device, the monitoring object person information detected by the sensor device, and determines an abnormal state of the monitoring object person based on the monitoring object person information; and
a plurality of terminal devices communicably connected to the central processing device, and
the method includes:
   an acquisition step of acquiring, from among the plurality of terminal devices, a notification destination terminal device which is a terminal device of a notification destination to be notified of abnormality of the monitoring object person, and is determined in accordance with an abnormal state of the monitoring object person determined by the central processing device;
   an abnormality notification step of notifying, of the abnormality of the monitoring object person, the notification destination terminal device acquired in the acquisition step; and
   an information transmission step of transmitting the monitoring object person information to the notification destination terminal device from the central processing device in response to a transmission request for the monitoring object person information from the notification destination terminal device that has been notified of abnormality of the monitoring object person in the abnormality notification step.

Advantages and characteristics provided by one or more embodiments of the invention are fully understood from the detailed description provided hereunder and the accompanying drawings. The detailed description and the accompanying drawings are provided as only examples and not intended as definitions to limit the present invention.

### Brief Description of Drawings

Fig. 1 is a diagram illustrating a configuration of an assistance system in the present embodiment.
Fig. 2 is a diagram illustrating a configuration of a management server device in the assistance system.
Fig. 3 is a diagram illustrating a configuration of a first server-side monitoring information table.
Fig. 4 is a diagram illustrating a configuration of a second server-side monitoring information table.
Fig. 5 is a diagram illustrating a configuration of terminal device information.
Fig. 6 is a diagram illustrating a configuration of a server-side sensor information table.
Fig. 7 is a diagram illustrating a configuration of a mobile terminal device in the assistance system.
Fig. 8 is a sequence diagram schematically illustrating exemplary operation in the assistance system.
Fig. 9 is a diagram illustrating an exemplary notification screen.
Fig. 10 is a view illustrating an exemplary user list selection screen.
Fig. 11 is a view illustrating an exemplary personal identification number input screen.
Fig. 12 is a view illustrating an exemplary monitoring information screen displayed on the mobile terminal device.
Fig. 13 is a view illustrating an exemplary personalized vital sign information display screen.
Fig. 14 is a view illustrating an exemplary personalized vital sign information display screen.
Fig. 15 is a view illustrating an exemplary personalized vital sign information display screen.
Fig. 16 is a view illustrating an exemplary personalized vital sign information display screen.
Fig. 17 is a view illustrating an exemplary submenu screen displayed on the mobile terminal device.
Fig. 18 is a view illustrating an exemplary format selection screen displayed on the mobile terminal device.
Fig. 19 is a view illustrating an exemplary recording format screen displayed on the mobile terminal device.
Fig. 20 is a sequence diagram schematically illustrating exemplary operation different from the operation in Fig. 8.
Fig. 21 is a sequence diagram schematically illustrating exemplary operation different from operation in Fig. 8 and Fig. 20.

### Description of Embodiments

### (Underlying Knowledge Forming Basis of Present Invention)

First, underlying knowledge forming the basis of the present invention will be described. As described above, in the abnormality notification device disclosed in above Patent Literature 1, when an abnormality of a patient is detected, a notification destination to be notified of the abnormality of the patient is changed between a case where reliability of an abnormality detection result is high and a case where the reliability is low. However, speaking of an abnormality of a monitoring object person such as a patient, there are various states of abnormalities.

For example, in a case where a respiration rate of a monitoring object person is increased more than usual, the person may have a fever, and therefore, it can be considered that it is more preferable to notify a nurse rather than a care worker. On the other hand, in a case where a monitoring object person falls from a bed, it can be considered that it is preferable to notify a care worker rather than a nurse. Based on such underlying knowledge, the inventors of the present invention have come to conceive the invention in which a notification destination to be notified of an abnormality of a monitoring object person is determined in accordance with an abnormal state of the monitoring object person.

### (Embodiments)

Hereinafter, one or more embodiments of the present invention will be described with reference to the drawings. However, the scope of the invention is not limited to the disclosed embodiments. Note that, in the drawings, elements denoted by the same reference sign represent the identical elements, and a description thereof will be omitted as appropriate. In the present specification, in a case of generically naming an element, the element will be denoted by a reference sign without a suffix, and in a case of individually naming an element, the element will be denoted by a reference sign with a suffix.

An assistance system that assists monitoring a monitoring object person in the present embodiment assists monitoring a monitoring object person Ob who is a monitoring target to be monitored. Note that a monitoring object person Ob can also be determined as a watching target person who is a watching target to be watched. The assistance system of the present embodiment includes a sensor device, a management server device, a fixed terminal device, and a plurality of mobile terminal devices. The sensor device detects vital sign information and the like of the monitoring object person Ob and transmits detection data to the management server device. Each of the plurality of mobile terminal devices is carried around by a monitoring person (user) such as a nurse, a care worker, a doctor, or the like.

The management server device includes a communicator that performs communication and is communicably connected to the sensor device and each of the plurality of mobile terminal devices. When detection data is received from the sensor device, the management server device determines whether a monitoring object person Ob is in an abnormal state. In a case of determining that the monitoring object person Ob is in the abnormal state, the management server device ranks the plurality of mobile terminal devices and determines a notification destination to be notified of the fact that the monitoring object person Ob is in the abnormal state. The management server device notifies the determined notification destination mobile terminal device of the fact that the monitoring object person Ob is in the abnormal state.

In the present embodiment, the notification destination mobile terminal device is operated by a user to request the management server device to transmit the vital sign information and the like of the monitoring object person Ob. In response to the request from the notification destination mobile terminal device, the management server device transmits latest vital sign information and the like of the monitoring object person Ob to the notification destination mobile terminal device.

### (Configuration)

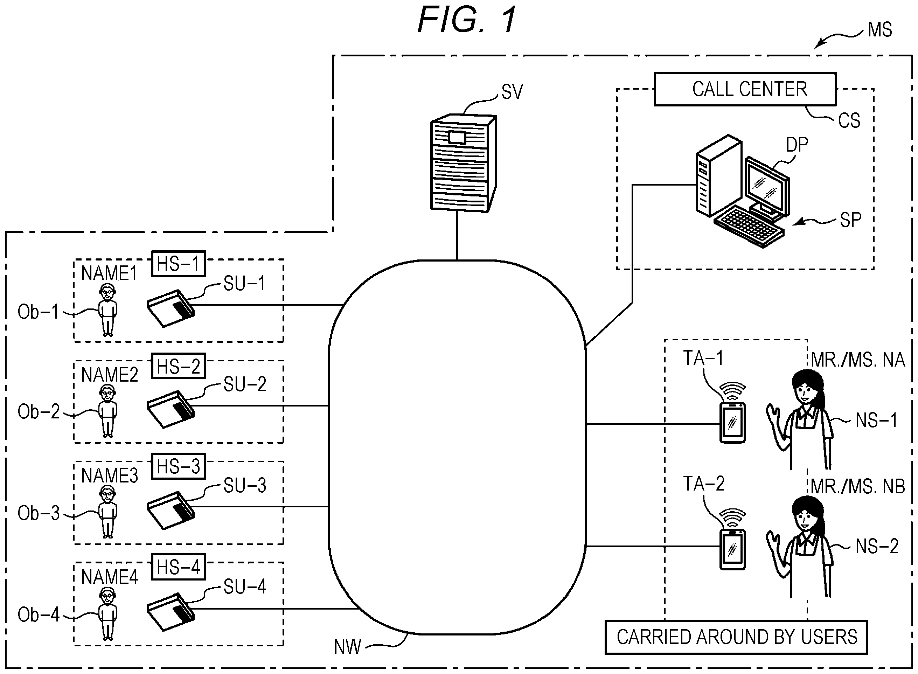

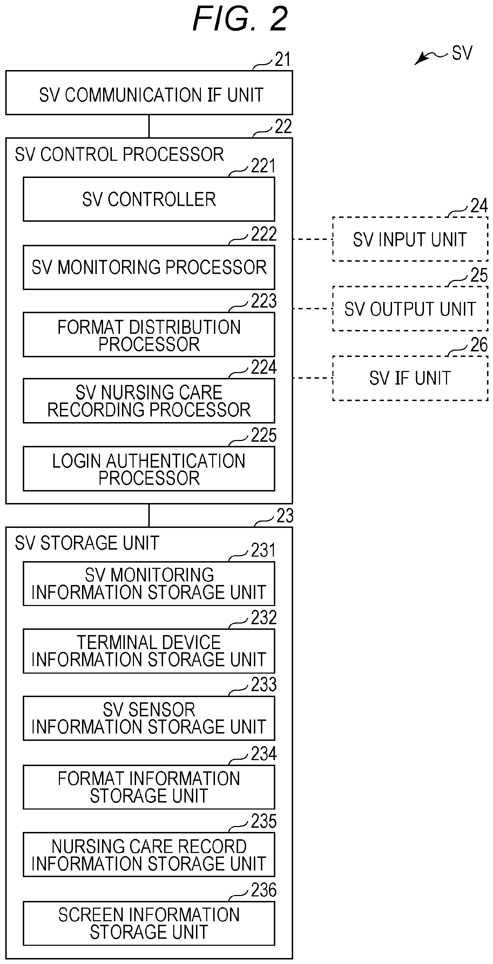

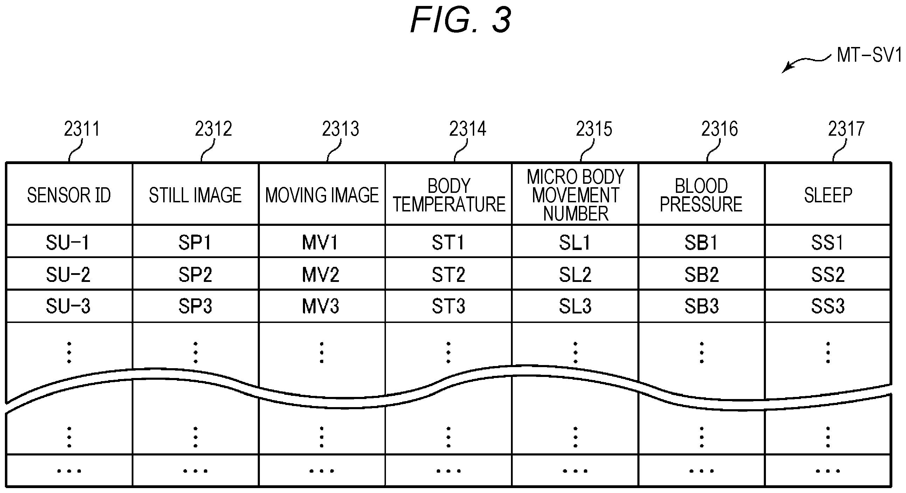

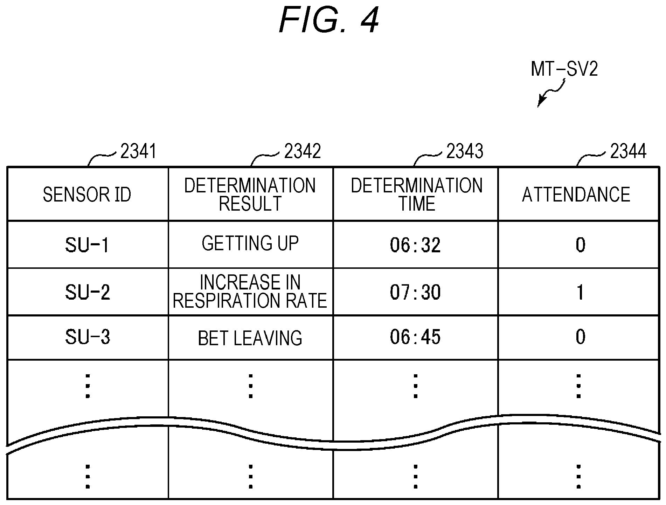

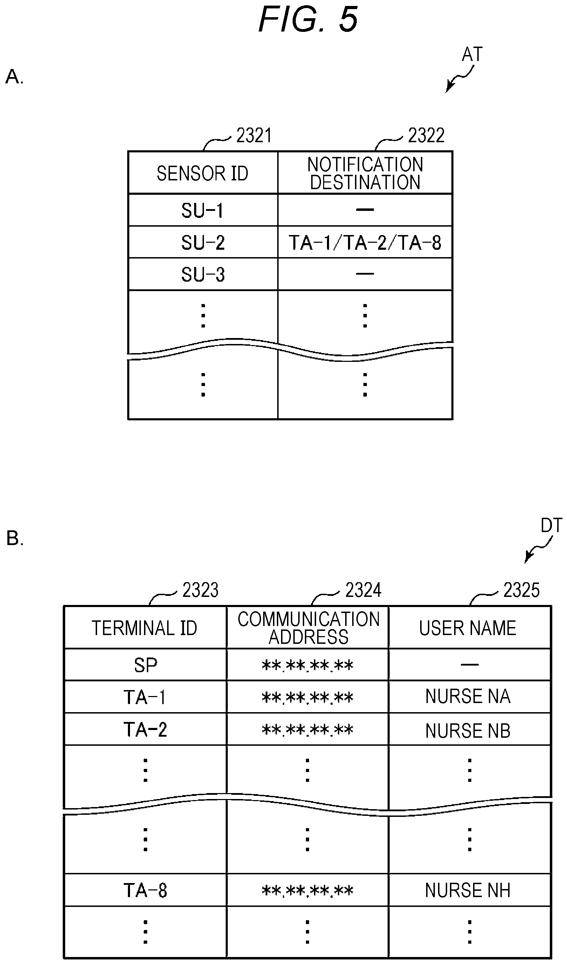

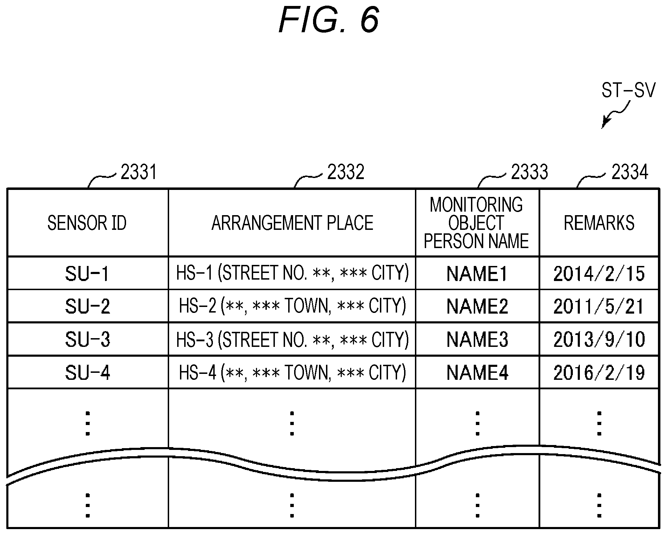

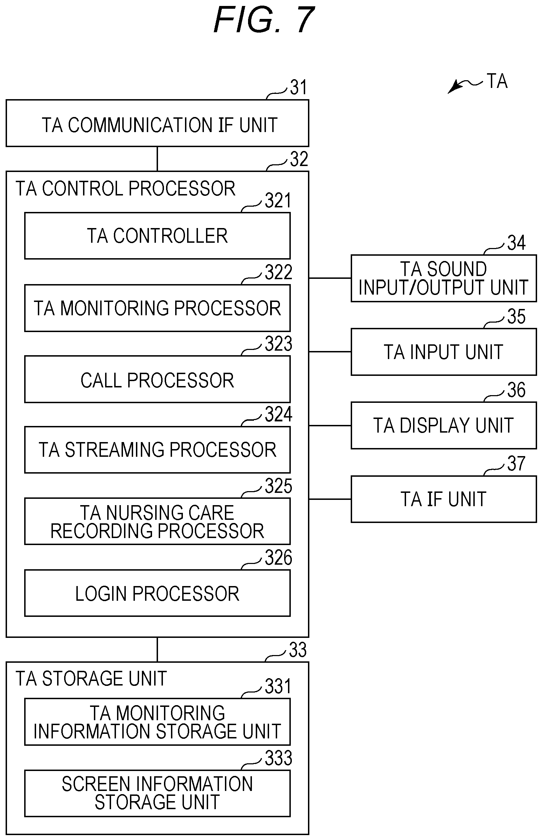

Fig. 1 is a diagram illustrating a configuration of the assistance system in the present embodiment. Fig. 2 is a diagram illustrating a configuration of the management server device in the assistance system of Fig. 1. Fig. 3 is a diagram illustrating a configuration of a first server-side monitoring information table stored in the management server device of Fig. 2. Fig. 4 is a diagram illustrating a configuration of a second server-side monitoring information table stored in the management server device of Fig. 2. Fig. 5 is a diagram illustrating a configuration of terminal device information stored in the management server device of Fig. 2. Fig. 5A illustrates a configuration of a notification destination information table of the terminal device information, and Fig. 5B illustrates a configuration of a terminal information table of the terminal device information. Fig. 6 is a diagram illustrating a configuration of a server-side sensor information table stored in the management server device of Fig. 2. Fig. 7 is a diagram illustrating a configuration of a mobile terminal device in the assistance system of Fig. 1.

More specifically, as illustrated in Fig. 1, an assistance system MS in the present embodiment includes, for example, one or a plurality of sensor devices SU (SU-1 to SU-4), a management server device SV, a fixed terminal device SP, and a plurality of mobile terminal devices TA (TA-1 and TA-2), which are communicably connected to one another by radio via a network NW In the example illustrated in Fig. 1, the network NW includes, for example, a mobile phone network and the Internet. The plurality of sensor devices SU-1 to SU-4, the management server device SV, the fixed terminal device SP, and the plurality of mobile terminal devices TA-1 and TA-2 may also be respectively connected to the network NW via an access point (not illustrated).

The assistance system MS is arranged in an appropriate place in accordance with a monitoring object person Ob. The monitoring object person Ob is, for example, a person requiring nursing due to illness, injury, or the like, a person requiring care due to decline in physical ability, or the like, a solitary person who lives alone, or the like. Particularly, from the viewpoint of achieving early discovery and early treatment, in a case where a predetermined unfavorable event such as an abnormal state happens to a monitoring object person Ob, the monitoring object person is preferably a person who requires discovery of such a state. For this reason, the assistance system MS is preferably arranged in a building such as a hospital, an elderly welfare facility, a dwelling, or the like, in accordance with a type of the monitoring object person Ob. In the example illustrated in Fig. 1, each of a plurality of monitoring object persons Ob is a solitary person who lives alone. Additionally, the fixed terminal device SP is arranged in a call center CS.

Each sensor device SU is arranged in a house HS of each monitoring object person Ob. The sensor device SU has a communication function to communicate with the management server device SV via the network NW, detects information associated with the monitoring object person Ob, and transmits the detection data to the management server device SV via the network NW. Examples of the sensor device SU include a Doppler sensor, an image sensor, a blood-pressure gauge, a thermometer, and the like.

More specifically, the examples of the sensor device SU include: a communication interface circuit to communicate with other devices SV, SP, and TA via the network NW; a nurse call processing circuit to receive a nurse call and notify the management server device SV; a call processing circuit to perform voice call with the mobile terminal device TA; an information transmission processing circuit to transmit output of the Doppler sensor, the image sensor, the blood-pressure gauge, and the thermometer to the predetermined other device SV, SP, or TA; a control circuit to control these; and a peripheral circuit thereof. At the time of transmitting detection data to the management server device SV via the network NW, the sensor device SU transmits the detection data together with a sensor ID that identifies an own device.

The Doppler sensor is arranged, for example, in a bedroom of each monitoring object person Ob. The Doppler sensor transmits ultrasonic waves or microwave transmission waves, receives reflection waves of the transmission waves reflected at an object, and outputs a Doppler signal of a Doppler frequency component based on the transmission waves and the reflection waves. The Doppler sensor transmits the transmission waves to, for example, the bedroom of the monitoring object person Ob. In a case where the object is moving, since a frequency of the reflection waves is shifted in proportion to a moving speed of the object due to a so-called Doppler effect, a difference (Doppler frequency component) is generated between the frequency of the transmission waves and the frequency of the reflection waves. The Doppler sensor generates and outputs a signal of the Doppler frequency component as a Doppler signal. The sensor device SU transmits the Doppler signal output from the Doppler sensor to the management server device SV via the network NW.

The image sensor is arranged in a bedroom of each house HS, for example. The image sensor has a configuration such that an imaging range of the image sensor is the entire bedroom, for example. The image sensor captures an image of the imaging range (namely, the entire bedroom) every predetermined period (e.g., 1/60 seconds), for example. The image may include a still image and a moving image. The sensor device SU transmits the imaging signal captured by the image sensor to the management server device SV via the network NW every predetermined period (e.g., 1/60 seconds). Note that the image sensor may also be provided in a room other than the bedroom of the house HS.

Additionally, when the monitoring object person Ob puts the blood-pressure gauge on his/her body by himself/herself and measures a blood pressure and a pulse, the sensor device SU transmits blood pressure data and pulse data to the management server device SV via the network NW. Additionally, when the monitoring object person Ob measures a body temperature by using the thermometer by himself/herself, the sensor device SU transmits body temperature data to the management server device SV via the network NW. The sensor device SU may further include a pulse oximeter. When the monitoring object person Ob puts the pulse oximeter on his/her body by himself/herself, the sensor device SU may transmit a measured blood oxygen saturation level to the management server device SV via the network NW.

In Fig. 1, for example, four sensor devices including first to fourth sensor devices SU-1 to SU-4 are illustrated, and the first sensor device SU-1 is arranged in a house HS-1 of a Mr./Ms. NAME1 Ob-1 who is one of monitoring object persons Ob, the second sensor device SU-2 is arranged in a house HS-2 of Mr./Ms. NAME2 Ob-2 who is one of the monitoring object persons Ob, the third sensor device SU-3 is arranged in a house HS-3 of Mr./Ms. NAME3 Ob-3 who is one of the monitoring object persons Ob, and the fourth sensor device SU-4 is arranged in a house HS-4 of Mr./Ms. NAME4 Ob-4 who is one of the monitoring object persons Ob.

The management server device SV (exemplary central processing device) has a communication function to communicate with the other devices SU, TA, and SP via the network NW, and manages the entire assistance system MS. As illustrated in Fig. 2, for example, the management server device SV includes: a server-side communication interface unit (SV communication IF unit) 21; a server-side control processor (SV control processor) 22; and a server-side storage unit (SV storage unit) 23.

The SV communication IF unit 21 is connected to the SV control processor 22 and is a communication circuit to perform communication under the control of the SV control processor 22. The SV communication IF unit 21 generates, in accordance with a communication protocol used in the network NW of the assistance system MS, a communication signal containing data which is fed from the SV control processor 22 and should be transferred, and then transmits the generated communication signal to the other devices SU, SP and TA via the network NW. The SV communication IF unit 21 receives a communication signal from each of the other devices SU, SP, and TA via the network NW, retrieves data from the received communication signal, converts the retrieved data to data that can be processed by the SV control processor 22, and outputs the converted data to the SV control processor 22. The SV communication IF unit 21 may include a communication interface circuit conforming to the IEEE 802.11 standards or the like, and may be connected to the network NW via a wireless LAN.

The SV storage unit 23 is connected to the SV control processor 22 and is a circuit that stores various kinds of predetermined programs and various kinds of predetermined data under the control of the SV control processor 22. The various kinds of predetermined programs include, for example, control processing programs such as: an SV control program to control the respective components of the management server device SV in accordance with the functions of the respective components; an SV monitoring processing program to execute predetermined information processing associated with monitoring for each monitoring object person Ob; a format distribution processing program to transmit, as a reply, a requested recording format to a mobile terminal device TA that is a transmission source when a request for the recording format is received from the mobile terminal device TA; a SV nursing care record processing program to store a nursing care record when the nursing care record using the recording format is received; and a login authentication processing program to authenticate login of each mobile terminal device TA.

The various kinds of predetermined data include necessary data to execute the respective programs, such as: a server identifier (server ID) of an own device to specify the management server device SV and identify the management server device SV; monitoring information associated with monitoring for each monitoring object person Ob; terminal device information representing information of each notification destination mobile terminal device TA to be notified of an abnormality of a monitoring object person; sensor information associated with a sensor device SU; format information associated with a recording format; nursing care record information that is information of a nursing care record; and screen information representing a display screen.

The SV storage unit 23 includes, for example, a read only memory (ROM) that is a nonvolatile storage element, an electrically erasable programmable read only memory (EEPROM) that is a rewritable nonvolatile storage element, and the like. The SV storage unit 23 includes a random access memory (RAM) serving as a working memory of the so-called SV control processor 22 that stores data and the like generated during execution of the predetermined programs, and the like.

Additionally, to store the monitoring information, the terminal device information, the sensor information, the format information, the nursing care record information, and the screen information respectively, the SV storage unit 23 functionally includes: a server-side monitoring information storage unit (SV monitoring information storage unit) 231; a terminal device information storage unit 232; a server-side sensor information storage unit (SV sensor information storage unit) 233; a format information storage unit 234; a nursing care record information storage unit 235; and a screen information storage unit 236.

The SV monitoring information storage unit 231 stores monitoring information of each monitoring object person Ob received from each sensor device SU, a determination result and the like of a state of each monitoring object person Ob determined based on the received monitoring information. In the present embodiment, the monitoring information of each monitoring object person Ob and a determination result of the state of each monitoring object person Ob are stored in a table form in the SV monitoring information storage unit 231.

As illustrated in Fig. 3, the first server-side monitoring information table (SV monitoring information table) MT-SV1 that registers the monitoring information associated with monitoring object persons Ob includes, for example: a sensor ID field 2311 that registers a sensor ID stored in a communication signal received from each of the sensor devices SU; a still image field 2312 that registers a still image contained in a communication signal received from each of the sensor devices SU; a moving image field 2313 that registers a moving image contained in a communication signal received from each of the sensor devices SU: a body temperature field 2314 that registers body temperature data contained in a communication signal received from each of the sensor devices SU; a micro body movement number field 2315 that registers the number of micro body movements contained in a communication signal received from each of the sensor devices SU; a blood pressure field 2316 that registers blood pressure data contained in a communication signal received from each of the sensor devices SU; and a sleep field 2317 that registers a sleep state contained in a communication signal received from each of the sensor devices SU. For example, each data may be registered in each of the fields 2312 to 2317, and a file name of each data may be registered, for example. In Fig. 3, a file name of each data is registered.

As illustrated in Fig. 4, the second server-side monitoring information table (SV monitoring information table) MT-SV2 that registers determination results of states of the respective monitoring object persons Ob includes, for example: a sensor ID field 2341 that registers a sensor ID corresponding to each of the determination results; a determination result field 2342 that registers a determination result of a state of each of the monitoring object persons Ob determined based on monitoring information; a determination time field 2343 that registers determination time at which the state of each of the monitoring object person Ob is determined; and an attendance field 2344 that registers necessity of attendance for the determination result of the state of each of the monitoring object person Ob, and a record is provided per determination result.

In the attendance field 2344, as described later, an attendance flag indicating necessity of attendance for each of the monitoring object persons Ob based on the determination result of the state of each of the monitoring object persons Ob determined based on the monitoring information. For example, in the present embodiment, an attendance flag "1" indicating necessity of attendance for a monitoring object person Ob or an attendance flag "0" indicating unnecessity of attendance for a monitoring object person Ob is registered in the attendance field 2344. Note that, as default, the attendance flag "0" indicating unnecessity of attendance for a monitoring object person Ob is registered in the attendance field 2344.

The terminal device information storage unit 232 stores information of a notification destination mobile terminal device determined in accordance with a determination result of a state of each monitoring object person Ob in a case where attendance for the monitoring object person Ob is necessary based on the determination result of the state of the monitoring object person Ob determined based on the monitoring information, Additionally, the terminal device information storage unit 232 stores information indicating a correspondence relation among a terminal ID of the fixed terminal device SP/a terminal ID of each mobile terminal device TA, a communication address of each thereof, and a monitoring person (user) using each mobile terminal devices TA. The terminal IDs are identifiers to identify the fixed terminal device SP and each of the mobile terminal devices TA.

In the present embodiment, each of the above-described information is stored in the table form in the terminal device information storage unit 232. For example, as illustrated in Fig. 5A, a notification destination information table AT that registers the information of the notification destination mobile terminal devices includes: a sensor ID field 2321 that registers the sensor IDs of the sensor devices SU having transmitted monitoring information; and a notification destination field 2322 that registers the terminal IDs of the notification destination mobile terminal devices TA, and a record is provided per sensor ID.

As illustrated in Fig. 5B, for example, a terminal information table DT that registers information indicating a correspondence relation between each terminal ID, a communication address thereof, and a monitoring person (user) using the mobile terminal devices TA includes: a terminal ID field 2323 that registers the terminal ID of fixed terminal devices SP and terminal IDs of the mobile terminal devices TA; a communication address field 2324 that registers a communication address of the fixed terminal device SP and communication addresses of the mobile terminal devices TA corresponding to the respective terminal IDs registered in the terminal ID field 2323; and a user name field 2325 that registers names of monitoring persons (users) using the mobile terminal devices TA corresponding to the respective terminal IDs registered in the terminal ID field 2323, and a record is provided per the terminal ID.

Note that the sensor IDs, the server IDs, and the terminal IDs may be respectively serial numbers each including a predetermined symbol string, for example, or may be communication addresses, for example. In a case where the terminal IDs are the communication addresses, the communication address field 2324 in Fig. 5B can be omitted.

Additionally, when a determination result registered in the determination result field 2342 (Fig. 4) of the second SV monitoring information table MT-SV2 is changed, a notification destination registered in the notification destination field 2322 of the notification destination information table AT is also changed.

The SV sensor information storage unit 233 stores sensor information associated with the sensor devices SU. In the present embodiment, the SV sensor information storage unit 233 stores, as the sensor information, information in which a sensor ID of each sensor device SU and a monitoring object person name of each monitoring object person Ob are correlated to each other.

In the present embodiment, such sensor information is stored in the table form in the SV sensor information storage unit 233. More specifically, as illustrated in Fig. 6, a server-side sensor information table (SV sensor information table) ST-SV that registers the sensor information includes, for example: a sensor ID field 2331 that registers the sensor IDs; an arrangement place field 2332 that registers arrangement places of the sensor devices SU having the IDs registered in the sensor ID field 2331; a monitoring object person name field 2333 that registers the monitoring object persons names of the monitoring object persons Ob to be monitored by the sensor devices SU having the sensor IDs registered in the sensor ID field 2331 (in other words, the monitoring object persons Ob who are present in the arrangement places of the sensor devices SU having the sensor IDs registered in the sensor ID field 2331); a remarks field 2334 that registers remarks associated with the sensor devices SU having the sensor IDs registered in the sensor ID field 2331, the arrangement places thereof, and the monitoring object persons Ob thereof, and a record is provided per sensor ID (in other words, sensor device SU). Note that, in Fig. 6, dates when the respective sensor devices SU having the sensor IDs registered in the sensor ID field 2331 are arranged in the arrangement places registered in the arrangement place field 2332 are registered in the remarks field 2334.

The format information storage unit 234 stores format information associated with a plurality of recording formats in accordance with kinds of nursing care including at least one of nursing and care. The format information is information associated with the recording formats to input content of the nursing care as a nursing care record. A plurality of recording formats is prepared in advance as the predetermined formats (formats, input forms) in accordance with the kinds of the nursing care.

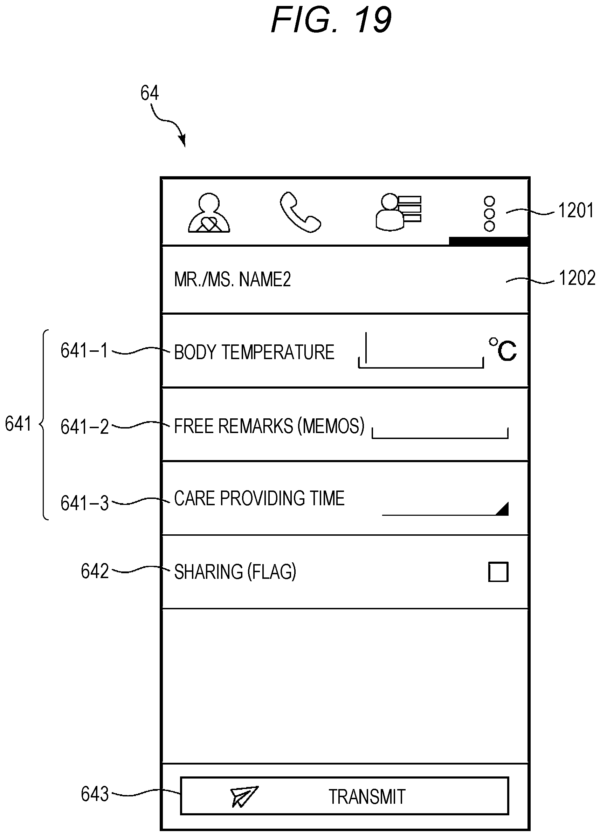

There are various kinds of nursing care in accordance with actual practice of nursing, actual practice of care, and the like. For example, the kinds of nursing care can include periodic care, care depending on a place, and the like. More specifically, the periodic care can include morning care (changing clothes, washing a face, attaching a denture, and the like), meal care (breakfast care, lunch care, dinner care), snack care, medication care, tooth brushing care, rehydration care, vital check, evening care (changing clothes, tooth brushing, removing a denture, transfer to a bed, and the like), patrol, body position change, and the like, which are daily executed, as well as bath care, rehabilitation, recreation, home visit, body cleaning, weight measurement, and the like which are performed every certain day of the week or every certain date. The care depending on a location can include excretory care and the like. These kinds of nursing care depend on places such as a lounge, a living room RM, a toilet, a bath, and a nurse station, and also depend on monitoring persons such as a nurse, a care worker, and a person in charge of the nursing care. The recording format is prepared in advance in accordance with content of such nursing care and includes, for example, a recording format for the morning care, a recording format for the meal care, a recording format for the snack care, a recording format for the medication care, a recording format for the tooth brushing care, a recording format for the rehydration care, a recording format for the vital check (see Fig. 19 described later), and the like.

Each of the format information is stored in the format information storage unit 234 in a manner correlated to each kind of the nursing care. In the present embodiment, an electronic file of each of the recording formats is described in a markup language such as a hyper text markup language (HTML) or an extensible markup language (XML), and is stored in the format information storage unit 234.

The nursing care record information storage unit 235 stores the nursing care record information that is information of nursing care records. A monitoring object person name that is a target of nursing care and the content of nursing care record are correlated to each other and stored in the nursing care record information storage unit 235 as the nursing care record information.

The screen information storage unit 236 stores screen information representing a display screen displayed on each mobile terminal device TA. The screen information storage unit 236 preliminarily stores, for example, electronic files of a notification screen 900, a submenu screen 61, a format selection screen 63, and the like described later which are transmitted to the mobile terminal device TA and described later.

The SV control processor 22 controls the respective units of the management server device SV in accordance with the functions of the respective units, and manages the entire assistance system MS. The SV control processor 22 includes, for example, a central processing unit (CPU) and a peripheral circuit thereof. The CPU of the SV control processor 22 has a clock function. The SV control processor 22 functionally includes a server-side controller (SV controller) 221, a server-side monitoring processor (SV monitoring processor) 222, a format distribution processor 223, a server-side nursing care recording processor (SV nursing care recording processor) 224, and a login authentication processor 225, which are implemented by the CPU executing a control processing program stored in the SV storage unit 23. The login authentication processor 225 will be described later.

The SV controller 221 controls the respective units of the management server device SV in accordance with the functions of the respective units and conducts entire control for the management server device SV.

The SV monitoring processor 222 manages detection data transmitted from each sensor device SU (i.e., monitoring information associated with monitoring for each monitoring object person Ob). More specifically, the SV monitoring processor 222 performs following processing based on the monitoring information transmitted from each sensor device SU.

The SV monitoring processor 222 saves an imaging signal transmitted from each sensor device SU in the SV monitoring information storage unit 231. The SV monitoring processor 222 detects predetermined actions of each monitoring object person Ob based on the imaging signal transmitted from the sensor device SU.

In the present embodiment, the predetermined actions of each monitoring object person Ob include four actions, for example: getting up in which the monitoring object person Ob gets up; bed leaving in which the monitoring object person Ob leaves a bed; the monitoring object person Ob falling from the bed; and the monitoring object person Ob collapsing. The SV monitoring processor 222 detects, for example, a head of the monitoring object person Ob based on an imaging signal transmitted from the sensor device SU, and detects getting up, bed leaving, collapsing, falling from the bed of the monitoring object person Ob based on a temporal change in a size of the detected head of the monitoring object person Ob.

More specifically, first, the SV monitoring information storage unit 231 preliminarily stores: a first threshold value Th1 to identify a location area of the bed, a head size in a lying posture, and a head size in a sitting posture within the location area of the bed; a second threshold value Th2 to identify whether the head size is a head size in a standing posture in a bedroom excluding the location area of the bed; and a third threshold value Th3 to identify whether the head size is the head size in the lying posture in the bedroom excluding the location area of the bed.

Then, the SV monitoring processor 222 extracts a moving object area as a person area of the monitoring object person Ob from an image represented by an imaging signal by, for example, a background subtraction method or a frame difference method, and extracts a head area of the monitoring object person Ob from the extracted moving object area by, for example, circular or elliptical Hough transform or by pattern matching using, for example, a head model prepared in advance, or by a neural network that has learned head detection, for example, and then detects getting up, bed leaving, collapsing, and falling based on a position and the size of the extracted head.

For example, in a case where the position of the extracted head is in the location area of the bed and the size of the extracted head is temporally changed to the size in the sitting posture from the size in the lying posture by using the first threshold value Th1, the SV monitoring processor 222 determines that the action is getting up, and detects the getting up. For example, in a case where the position of the extracted head is temporally changed from the location area of the bed to the outside of the location area of the bed and the size of the extracted head is temporally changed from a certain size to the size in the standing posture by using the second threshold value Th2, the SV monitoring processor 222 determines that the action is bed leaving, and detects the bed leaving.

For example, in a case where the position of the extracted head is temporally changed from the inside of the location area of the bed to the outside of the location area of the bed and the size of the extracted head is temporally changed from the certain size to the size in the lying posture by using the third threshold value Th3, the SV monitoring processor 222 determines that the action is falling, and detects the falling. For example, in a case where the position of the extracted head is inside the bedroom excluding the location area of the bed and the size of the extracted head is temporally changed from the certain size to the size in the lying posture by using the third threshold value Th3, the SV monitoring processor 222 determines that the action is collapsing, and detect the collapsing.

In a case where the position of the extracted head is within the location area of the bed and the size of the head is not temporarily changed from the size in the lying posture, the SV monitoring processor 222 determines that the monitoring object person Ob1 is sleeping.

The SV monitoring processor 222 processes a Doppler signal of a time space transmitted from each sensor device SU and calculates the number of micro body movements of each monitoring object person Ob. The number of micro body movements of the monitoring object person Ob corresponds to a respiration rate of the monitoring object person Ob. As described above, the Doppler sensor transmits transmission waves to the bed of the monitoring object person Ob, for example. Therefore, when the number of micro body movements of the monitoring object person Ob can be calculated, the SV monitoring processor 222 may determine that the monitoring object person Ob is sleeping.

The SV monitoring processor 222 determines, based on the monitoring information of the monitoring object person Ob, whether the monitoring object person Ob is in an abnormal state regarding which a notification should be given to a user using a mobile terminal device TA. In a case of determining that the monitoring object person Ob is in the abnormal state regarding which the notification should be given to a user using a mobile terminal device TA, the SV monitoring processor 222 determines a user of a mobile terminal device TA to be notified by, for example, ranking the users of the mobile terminal devices TA based on the state of the monitoring object person Ob.

For example, in a case where a sleep state of the monitoring object person Ob is reversed between daytime and night time as a result of detecting the sleep state of the monitoring object person Ob, the SV monitoring processor 222 determines that improvement of his/her lifestyle is necessary and determines that a notification should be given to a mobile terminal device TA used by a family member of the monitoring object person Ob.

For example, when the number of micro body movements (i.e., respiration rate) of a monitoring object person Ob is normal and an elapsed time reaches a predetermined period from previous sputum suction treatment, the SV monitoring processor 222 determines that the sputum suction treatment is necessary, and determines that a notification should be given to a mobile terminal device TA used by a care worker.

For example, in a case where the number of micro body movements (i.e., respiration rate) of the monitoring object person Ob is increased, the SV monitoring processor 222 determines that there is a possibility of a fever, and determines that a notification should be given to a mobile terminal device TA used by a nurse. Note that, for example, when the number of micro body movements (i.e., respiration rate) is increased by 10% from a maximum value in a variation range of a normal state, the SV monitoring processor 222 may determine that the number of micro body movements (i.e., respiration rate) is increased and abnormal.

For example, in a case where a body temperature measured by the monitoring object person Ob is higher by 0.5 or more degrees and less than 2 degrees from an upper limit value in variation of the normal state even though the number of micro body movements (i.e., respiration rate) of the monitoring object person Ob is normal, the SV monitoring processor 222 determines that direct care is necessary, and determines that a notification should be given to the mobile terminal device TA used by the nurse.

For example, in a case where the body temperature measured by the monitoring object person Ob is higher by 2 degrees or more from the upper limit value of the variation of the normal state even though the number of micro body movements (i.e., respiration rate) of the monitoring object person Ob is normal, the SV monitoring processor 222 determines that diagnosis by a doctor is necessary, and determines that a notification should be given to a mobile terminal device TA used by the doctor.

For example, when collapsing or falling of a monitoring object person Ob is detected, the SV monitoring processor 222 determines that direct care is necessary, and determines that a notification should be given to the mobile terminal device TA used by the care worker. For example, when getting up or bed leaving of a monitoring object person Ob is detected, the SV monitoring processor 222 determines that the monitoring object person Ob is not in an abnormal state and determines that notification to the mobile terminal device TA is not necessary.

In a case of settling that notification to the mobile terminal device TA is necessary, the SV monitoring processor 222 registers the attendance flag "1" in the attendance field 2344 of the SV monitoring information table MT-SV2, and in a case of settling that notification to the mobile terminal device TA is not necessary, the SV monitoring processor 222 registers the attendance flag "0" in the attendance field 2344.

When a request for the recording format for a nursing care record is received from the notification destination mobile terminal device TA determined by the SV monitoring processor 222, the format distribution processor 223 transmits, as a reply, the requested recording format to the notification destination mobile terminal device TA. More specifically, in a case where a communication signal containing an order to request a recording format is received at the SV communication IF unit 21, the format distribution processor 223 extracts the requested recording format from among the plurality of recording formats stored in the format information storage unit 234 and transmits, as a reply, a communication signal containing the extracted recording format to the mobile terminal device TA having requested the recording format at the SV communication IF unit 21.

The SV nursing care recording processor 224 controls input processing of content of nursing care performed by a monitoring person (user) using each mobile terminal device TA.

When a request for the format selection screen for a nursing care record is received from the notification destination mobile terminal device TA determined by the SV monitoring processor 222, the SV nursing care recording processor 224 transmits, as a reply, the requested format selection screen to the notification destination mobile terminal device TA. More specifically, in a case where a communication signal containing an order to request the format selection screen is received at the SV communication IF unit 21, the SV nursing care recording processor 224 extracts the requested format selection screen stored in the screen information storage unit 236 and transmits, as a reply, a communication signal containing the extracted format selection screen to the mobile terminal device TA having requested the format selection screen at the SV communication IF unit 21.

Additionally, when a nursing care record using the recording format is received from the mobile terminal device TA, the SV nursing care recording processor 224 stores the nursing care record in the nursing care record information storage unit 235.

Note that, as indicated by broken lines in Fig. 2, the management server device SV may further include, as necessary: a server-side input unit (SV input unit) 24 that is connected to the SV control processor 22 and used to input, for example, various commands, various data, and the like; a server-side output unit (SV output unit) 25 that outputs the various commands and the various data input in the SV input unit 24, various monitoring information associated with monitoring for each monitoring object person Ob, and the like; and a server-side interface unit (SV IF unit) 26 that performs data input/output with an external device.

The above management server device SV can be constituted of a computer having a communication function, for example.

The fixed terminal device SP is arranged in the call center CS. The fixed terminal device SP includes: a communication function to communicate with the other devices SU, SV, and TA via the network NW; a display unit DP to display predetermined information; an input function to input a predetermined instruction and data; and the like. The fixed terminal device SP functions as a user interface (UI) for a staff member in the call center CS by displaying, on the display unit DP, information associated with a notification destination mobile terminal device TA to be notified of abnormality of a monitoring object person Ob determined as abnormal by the SV monitoring processor 222 of the management server device SV. The above fixed terminal device SP can be constituted of a computer having a communication function, for example.

The mobile terminal device TA includes: a communication function to communicate with the management server device SV and each sensor device SU via the network NW; a display function to display predetermined information; an input function to input predetermined instructions and data; a call function to perform voice call, and the like. Each mobile terminal device TA is an apparatus to: input the predetermined instructions and the data to be provided to the management server device SV; display monitoring information generated based on detection data of each sensor device SU, in which the detection data is transmitted from the management server device SV; respond to a nurse call and talk through a voice call with each sensor device SU.

As illustrated in Fig. 7, such a mobile terminal device TA of the present embodiment includes, for example: a terminal-side communication interface unit (TA communication IF unit) 31; a terminal-side control processor (TA control processor) 32; a terminal-side storage unit (TA storage unit) 33; a terminal-side sound input/output unit (TA sound input/output unit) 34; a terminal-side input unit (TA input unit) 35; a terminal-side display unit (TA display unit) 36; and a terminal-side interface unit (TA IF unit) 37.

The TA sound input/output unit 34 is connected to the TA control processor 32 and includes a circuit to acquire an external sound and input the acquired sound to the mobile terminal device TA, and a circuit to generate and output a sound corresponding to an electric signal representing a sound under the control of the TA control processor 32. The TA sound input/output unit 34 includes, for example: a speaker or the like to convert an electric signal of a sound (sound data) into a mechanical vibration signal of the sound (sound signal); a microphone to convert a mechanical vibration signal of a sound in an audible range into an electric signal; and the like The TA sound input/output unit 34 outputs an electric signal representing the external sound to the TA control processor 32, converts an electric signal fed from the TA control processor 32 into a mechanical vibration signal of a sound, and outputs the mechanical vibration signal.

The TA input unit 35 is connected to the TA control processor 32, and includes, for example, a circuit to receive predetermined operation and input the operation to the mobile terminal device TA, and includes, for example, a plurality of input switches and the like in which predetermined functions are allocated. The predetermined operation includes, for example, various kinds of operation necessary to assist monitoring for each monitoring object person Ob such as: input operation of a user name and a personal identification number for login; request operation for a voice call and terminating operation thereof; request operation for a moving image live and terminating operation thereof; input operation on an "ATTEND" button to indicate an intention to provide attendance such as lifesaving, nursing, care, helping, or the like for a monitoring object person Ob regarding whom a notification has been provided; and input operation of a nursing care record by using a predetermined recording format.

The TA display unit 36 is connected to the TA control processor 32, and includes a circuit to: display predetermined operation content input from the TA input unit 35 under the control of the TA control processor 32, display monitoring information of each monitoring object person Ob (the number of micro body movements of the monitoring object person Ob calculated based on detection data by each sensor device SU, a sleep state of the monitoring object person Ob, a detected kind of the predetermined action, a nurse call received by the sensor device SU, images such as a still image and a moving image of the monitoring object person Ob, and the like) transmitted from the management server device SV; and display the recording format to input a nursing care record, and the like. For example, the TA display unit 36 is a display device such as a liquid crystal display (LCD) or an organic EL display.

Additionally, in the present embodiment, the TA input unit 35 and the TA display unit 36 constitute a touch panel. In this case, the TA input unit 35 is a position input device of a resistive film type, a capacitive type, or the like to perform inputting by detecting an operating position. In this touch panel, the position input device is provided on a display surface of the TA display unit 36, and candidates of one or a plurality of input items that can be input to the TA display unit 36 are displayed. When a monitoring person (user) such as a nurse or a care worker touches a display position where a desired input item is displayed, the position is detected by the position input device, and the display item displayed at the detected position is input to the mobile terminal device TA as the operation input item of the user.

The TA IF unit 37 is connected to the TA control processor 32 and includes a circuit that performs data input/output with an external device under the control of the TA control processor 32. The TA IF unit 37 may include, for example, an interface circuit or the like using the Bluetooth (registered trademark) standard, an interface circuit that performs infrared communication such as the IrDA standard, an interface circuit using the USB standard.

Similar to the SV communication IF unit 21, the TA communication IF unit 31 is connected to the TA control processor 32 and includes a communication circuit that performs communication under the control of the TA control processor 32. For example, the TA communication IF unit 31 may include a communication interface circuit conforming to the IEEE 802.11 standard or the like.

The TA storage unit 33 is connected to the TA control processor 32 and is a circuit that stores various predetermined programs and various kinds of predetermined data under the control of the TA control processor 32. Examples of the various predetermined programs include control processing programs such as: a TA control program to control the respective units of the mobile terminal device TA in accordance with the functions of the respective units; a TA monitoring processing program to execute predetermined information processing associated with monitoring for each monitoring object person Ob; a call processing program to perform a voice call with each sensor device SU by using the TA sound input/output unit 34 or the like; and a TA streaming processing program to display, on the TA display unit 36 by streaming reproduction, a moving image distributed from the management server device SV; a TA nursing care recording processing program to execute selection processing for a recording format and input processing of a nursing care record using the selected recording format; and a login processing program to execute login processing. The various kinds of predetermined data include data and the like necessary to execute the respective programs, such as an own device terminal ID, monitoring information of each monitoring object person Ob, and screen information displayed on the TA display unit 36.

The TA storage unit 33 includes, for example, a ROM, an EEPROM, or the like. The TA storage unit 33 includes a RAM or the like serving as a working memory of the so-called TA control processor 32 that stores data and the like generated during execution of the predetermined programs. The TA storage unit 33 functionally includes a terminal-side monitoring information storage unit (TA monitoring information storage unit) 331 and a screen information storage unit 333 to store the monitoring information and the screen information, respectively.

The TA monitoring information storage unit 331 temporarily stores the monitoring information transmitted from the management server device SV to display the monitoring information on the TA display unit 36.

The screen information storage unit 333 stores the screen information to be displayed on the TA display unit 36. The screen information storage unit 333 preliminarily stores, for example, electronic files of the notification screen 900, a user list selection screen 1000, the submenu screen 61, and the like, which are relatively frequently used and described later. The screen information storage unit 333 temporarily stores electronic files of the format selection screen 63, a recording format screen 64, and the like which are transmitted from the management server device SV and described later.

The TA control processor 32 controls the respective units of the mobile terminal device TA in accordance with the functions of the respective units. The TA control processor 32 includes, for example, a CPU and a peripheral circuit thereof. The TA control processor 32 functionally includes a terminal-side controller (TA controller) 321, a terminal-side monitoring processor (TA monitoring processor) 322, a call processor 323, a terminal-side streaming processor (TA streaming processor) 324, a terminal-side nursing care recording processor (TA nursing care recording processor) 325, and a login processor 326 which are implemented by the CPU executing the program stored in the TA storage unit 33. The login processor 326 will be described later.

The TA controller 321 controls the respective units of the mobile terminal device TA in accordance with the functions of the respective units and conducts entire control for the mobile terminal device TA.

The TA monitoring processor 322 is notified of abnormality of a monitoring object person Ob by the management server device SV, and when the login processing is performed, the TA monitoring processor 322 executes predetermined processing associated with attendance to the monitoring object person Ob. More specifically, the TA monitoring processor 322 communicates with the management server device SV and displays latest monitoring information of the monitoring object person Ob on the TA display unit 36. Then, predetermined input operation is received from the TA input unit 35, the TA monitoring processor 322 executes predetermined processing in accordance with the input operation. A specific processing procedure will be described later.

The call processor 323 performs voice call with each sensor device SU by using the TA sound input/output unit 34 and the like. More specifically, for example, the call processor 323 uses the TA sound input/output unit 34 and the like to perform voice call by a voice over internet protocol (VoIP) with the sensor device SU arranged in the house HS of the monitoring object person Ob who is in the abnormal state regarding which the notification has been provided from the management server device SV, for example.

The TA streaming processor 324 displays, on the TA display unit 36 by streaming reproduction, a moving image (e.g., a live moving image) distributed from the management server device SV and captured by the sensor device SU.

The TA nursing care recording processor 325 executes the input processing of a nursing care record using the predetermined recording format. When the user of the mobile terminal device TA attends to a monitoring object person Ob who is in the abnormal state, the TA nursing care recording processor 325 executes the input processing of a nursing care record in accordance with content of the attendance. The input processing of the nursing care record by the TA nursing care recording processor 325 will be described later.

Such a mobile terminal device TA can be constituted of a mobile communication terminal device such as a so-called tablet type computer, a smartphone, a cellular phone, or the like.

### (Operation)

Next, operation of the present embodiment will be described. In the assistance system MS having the above-described configuration, when power is turned on, the respective devices SU, SV, SP, and TA execute initialization required in the respective units, and start running. In the management server device SV, the SV controller 221, the SV monitoring processor 222, the format distribution processor 223, the SV nursing care recording processor 224, and the login authentication processor 225 are functionally implemented in the SV control processor 22 by executing the control processing program. In the mobile terminal device TA, the TA controller 321, the TA monitoring processor 322, the call processor 323, the TA streaming processor 324, the TA nursing care recording processor 325, and the login processor 326 are functionally implemented in the TA control processor 32 by executing the control processing program.

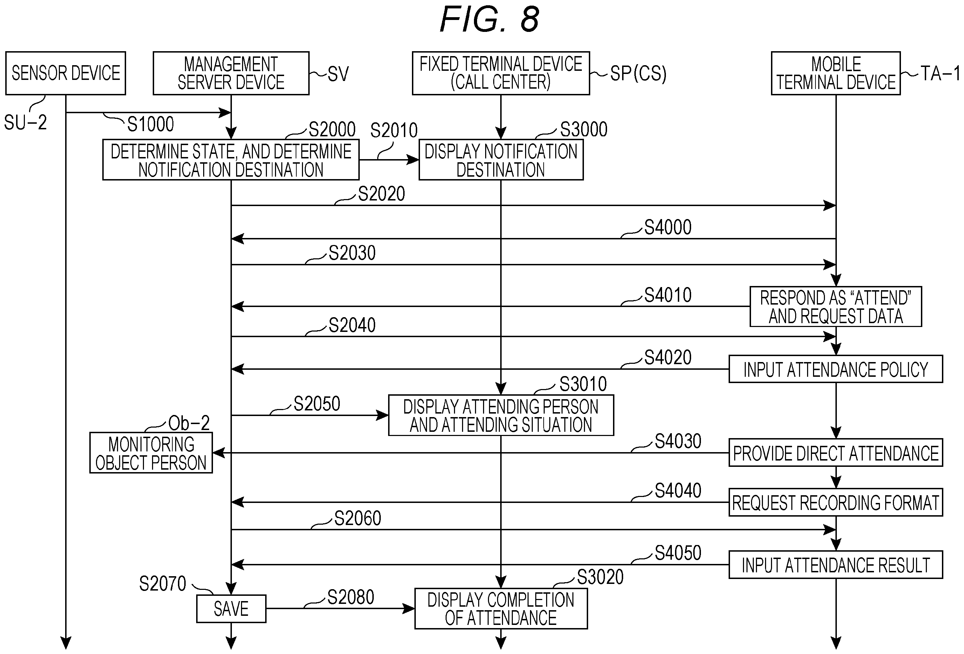

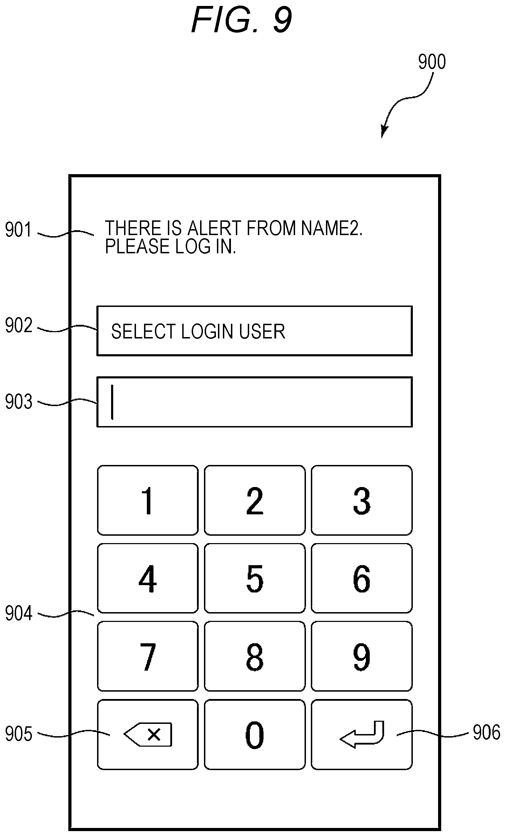

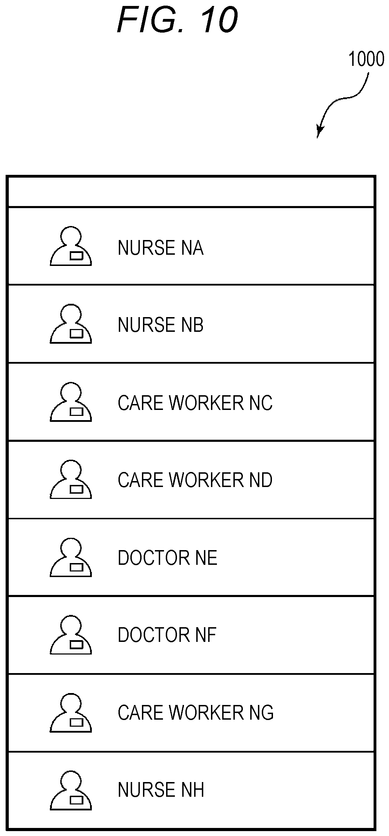

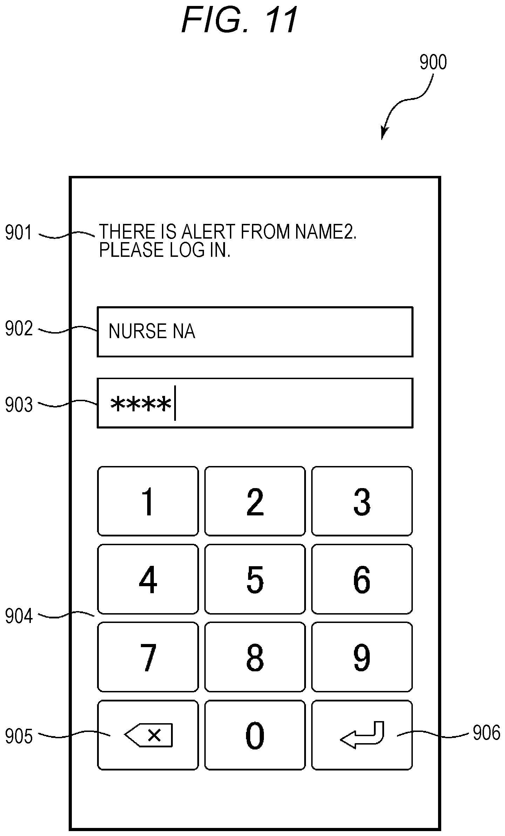

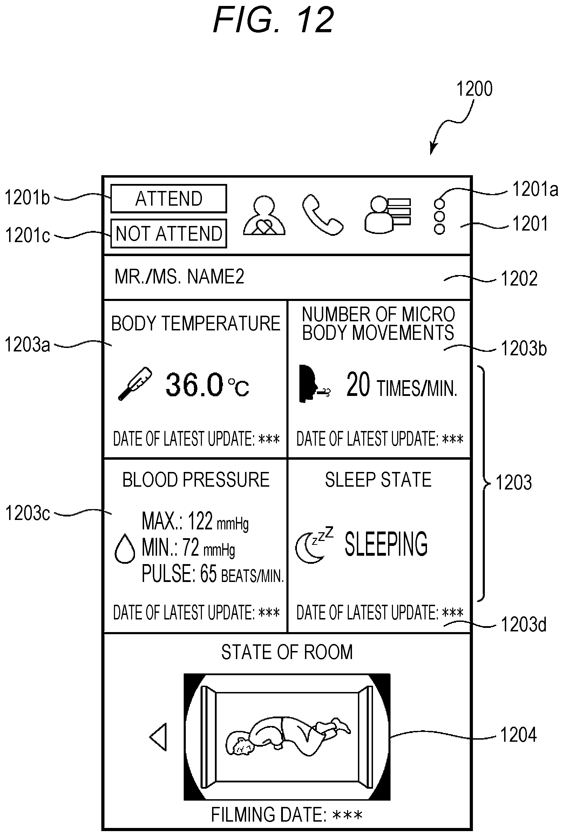

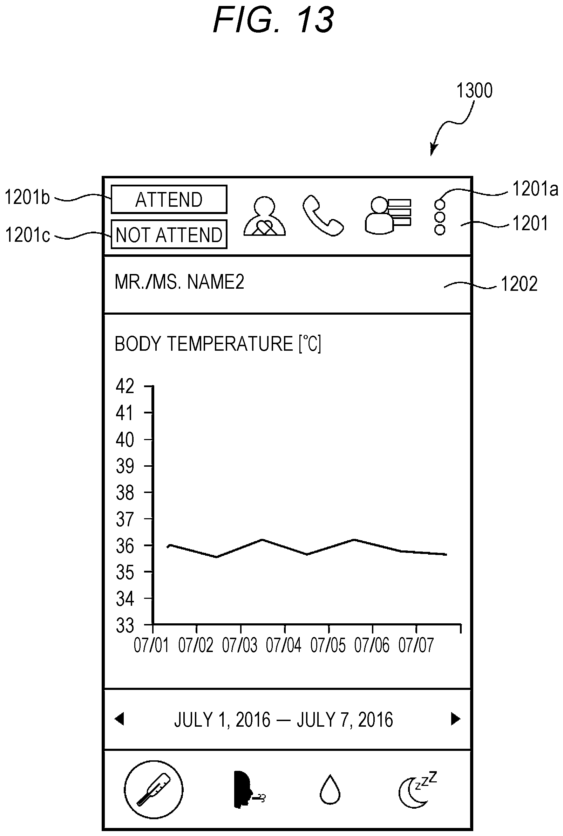

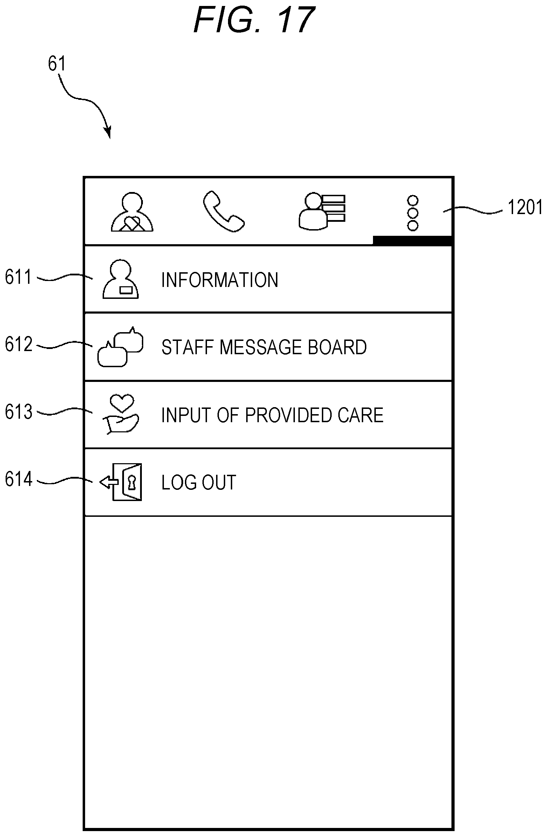

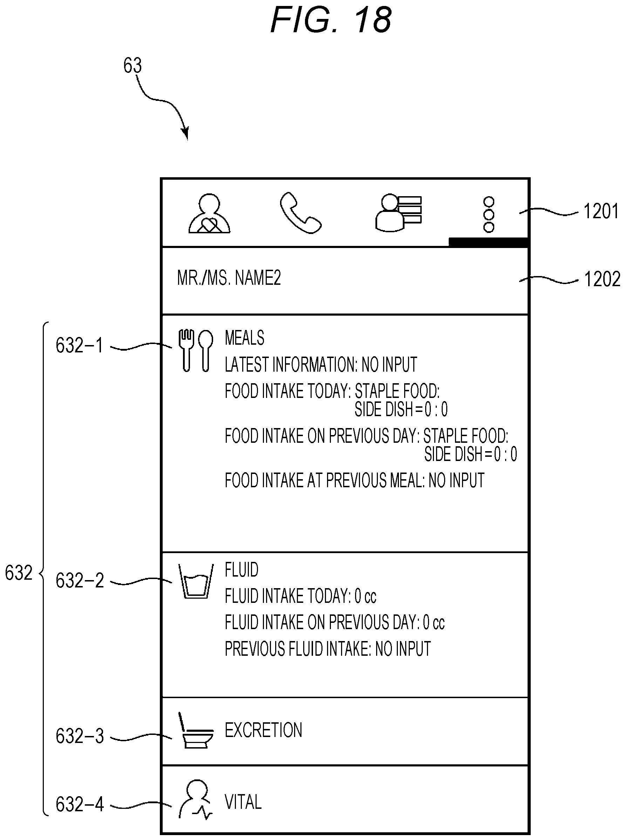

Fig. 8 is a sequence diagram schematically illustrating exemplary operation in the assistance system MS. Fig. 9 is a diagram illustrating an exemplary notification screen displayed on each mobile terminal device TA at the time of receiving a notification from the management server device SV. Fig. 10 is a view illustrating an exemplary user list selection screen displayed on the mobile terminal device TA. Fig. 11 is a view illustrating an exemplary personal identification number input screen displayed on the mobile terminal device TA. Fig. 12 is a view illustrating an exemplary monitoring information screen displayed on the mobile terminal device TA after completion of login authentication. Figs. 13 to 16 are views illustrating exemplary personalized vital sign information display screens displayed on the mobile terminal device TA, respectively. Fig. 17 is a view illustrating an exemplary submenu screen displayed on the mobile terminal device TA. Fig. 18 is a view illustrating an exemplary format selection screen displayed on the mobile terminal device TA. Fig. 19 is a view illustrating an exemplary recording format screen displayed on the mobile terminal device TA. Note that, in Fig. 8, an example of the sensor device SU-2 is illustrated among the plurality of sensor devices SU included in the assistance system MS.

In step S1000 of Fig. 8, detection data is transmitted from the sensor device SU-2 to the management server device SV. When the detection data is received, the SV monitoring processor 222 of the management server device SV determines a state of the monitoring object person Ob-2 based on the received detection data in step S2000. When it is determined that the monitoring object person Ob-2 is in a state regarding which a notification should be given to the mobile terminal device TA, the SV monitoring processor 222 determines a notification destination.

In Fig. 8, the SV monitoring processor 222 determines that a respiration rate of the monitoring object person Ob-2 is increased to 20 [times/min.]. In this case, as described above, the SV monitoring processor 222 determines that this is the state regarding which the notification should be given to the mobile terminal device TA, and then determines a nurse NA as the first, determines a nurse NB as the second, and determines a nurse NH as the third as ranking of notification destinations. The SV monitoring processor 222 refers to the terminal information table DT (Fig. 5B) in the terminal device information storage unit 232 to acquire the mobile terminal devices TA-1, TA-2, and TA-8 corresponding to the nurses NA, NB, and NH, and registers "TA-1/TA-2/TA-8" in the notification destination field 2322 of the notification destination information table AT (Fig. 5A) in the terminal device information storage unit 232.

Furthermore, in step S2000 (an exemplary acquisition step), the SV monitoring processor 222 refers to the terminal information table DT (Fig. 5B) in the terminal device information storage unit 232 and reads a communication address of the mobile terminal device TA-1. The SV monitoring processor 222 notifies the SV communication IF unit 21 of a fact that the mobile terminal device TA-1 is determined as a notification destination for the abnormality. The SV communication IF unit 21 acquires the communication address of the mobile terminal device TA-1 from the SV monitoring processor 222.

In step S2010, the SV communication IF unit 21 transmits, to the fixed terminal device SP, the notification destination determined by the SV monitoring processor 222. In step S3000, the fixed terminal device SP displays, on the display unit DP, the notification destination transmitted from the management server device SV.

In step S2020 (an exemplary abnormality notification step), the SV communication IF unit 21 notifies, of the abnormality of the monitoring object person Ob-2, the communication address of the mobile terminal device TA-1 acquired from the SV monitoring processor 222. With receipt of this notification, the login processor 326 of the mobile terminal device TA-1 displays, on the TA display unit 36 of the mobile terminal device TA-1, the notification screen 900 stored in the screen information storage unit 333, as illustrated in Fig. 9. However, information transmitted from the management server device SV is used for "NAME2" in a message column 901 described below.

The notification screen 900 includes: a message column 901 in which "THERE IS ALERT FROM NAME2. PLEASE LOG IN." is displayed; a user selection column 902 in which "SELECT LOGIN USER"; a personal identification number input column 903; a numeric key pad 904; an erase button 905; and a new line button 906. Note that the "NAME2" is a name of the monitoring object person Ob-2 who lives in the house HS-2 where the sensor device with the sensor ID "SU-2" is arranged as it can be grasped from the SV sensor information table ST-SV illustrated in Fig. 6 or from Fig. 1.

When input operation (tapping) is performed on the user selection column 902 by the user in a state in which the notification screen 900 is displayed on the TA display unit 36, the login processor 326 displays, on the TA display unit 36, the user list selection screen 1000 stored in the screen information storage unit 333 of the TA storage unit 33. As illustrated in Fig. 10, the user list selection screen 1000 includes a list of monitoring persons (users) registered in the assistance system MS of the present embodiment.

When input operation (tapping) is performed on the own name (the nurse NA in the present embodiment) on the user list selection screen 1000, the own name is displayed in the place of the "SELECT LOGIN USER" of the user selection column 902 in the notification screen 900 in Fig. 9. In this state, when a personal identification number is input and input operation (tapping) is performed on the new line button 906 as illustrated in Fig. 11 by using the numeric key pad 904, the login processor 326 makes the TA communication IF unit 31 transmit, to the management server device SV, an inquiry signal including the personal identification number and the selected own name and inquires whether the personal identification number is correct.

When the SV communication IF unit 21 of the management server device SV receives the above personal identification number, the login authentication processor 225 collates the received personal identification number with a preliminarily-held personal identification number. When the collation result is successful, the login authentication processor 225 determines that the login authentication is normally completed.

Returning to Fig. 8, when it is determined that the login authentication is normally completed, the SV monitoring processor 222 of the management server device SV transmits the latest monitoring information of the monitoring object person Ob-2 to the mobile terminal device TA-1 in step S2030 (an exemplary information transmission step). As illustrated in Fig. 12, the TA monitoring processor 322 of the mobile terminal device TA-1 displays, on the TA display unit 36, a monitoring information screen 1200 representing the monitoring information of the monitoring object person Ob-2 transmitted from the management server device SV.

Note that, in step S2030, the information of the monitoring object person Ob-2 transmitted from the SV monitoring processor 222 to the mobile terminal device TA-1 is the information associated with information indicating that the abnormal state of the monitoring object person Ob-2 determined in step S2000. For example, in Fig. 8, since the SV monitoring processor 222 determines in step S2000 that the respiration rate of the monitoring object person Ob-2 is increased, the information of the monitoring object person Ob-2 transmitted to the mobile terminal device TA-1 by the SV monitoring processor 222 in step S2030 is vital sign information of the monitoring object person Ob-2 including the respiration rate (the number of micro body movements).

The monitoring information screen 1200 in Fig. 12 includes, in the order from the top: a menu bar area 1201; a user name display area 1202 displaying a user name; a vital sign information display area 1203 displaying the user's vital sign information; an image area 1204 indicating a state of a room where the room is present. The menu bar area 1201 includes a submenu button 1201a, an "ATTEND" button 1201b, and a "NOT ATTEND" button 1201c.

The vital sign information display area 1203 includes: a body temperature display area 1203a displaying a body temperature of a user; a micro body movement number display area 1203b displaying the number of the micro body movements of the user; a blood pressure display area 1203c displaying a blood pressure and a pulse of the user; and a sleep state display area 1203d displaying a sleep state of the user.

When the SV monitoring processor 222 transmits the vital sign information of the monitoring object person Ob-2 to the mobile terminal device TA-1, link destination information of corresponding vital sign information is included in each of the display areas 1203a, 1203b, 1203c, and 1203d included in the vital sign information display area 1203. Consequently, when input operation (tapping) is performed on each of the display areas 1203a, 1203b, 1203c, and 1203d on the monitoring information screen 1200 illustrated in Fig. 12, the operation content is transmitted from the mobile terminal device TA-1 to the management server device SV. When the operation content is received, the SV monitoring processor 222 of the management server device SV transmits a corresponding personalized vital sign information display screen to the mobile terminal device TA-1.

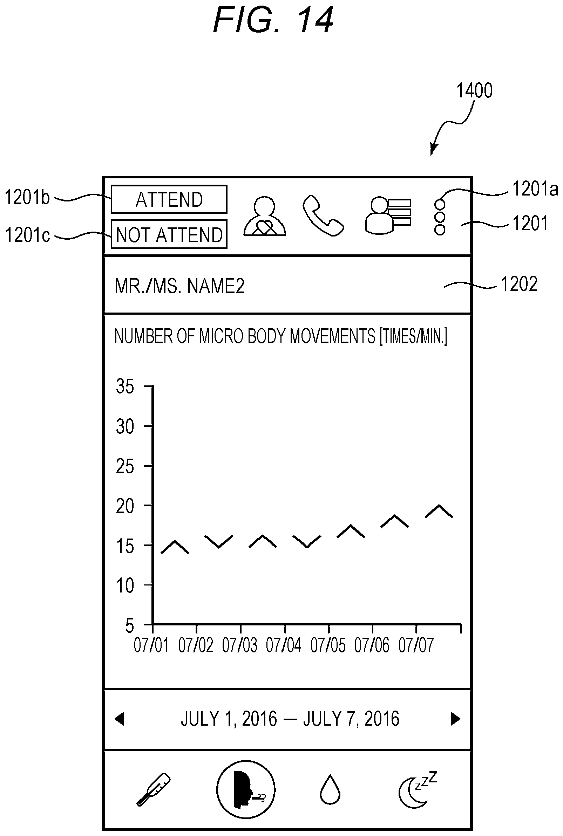

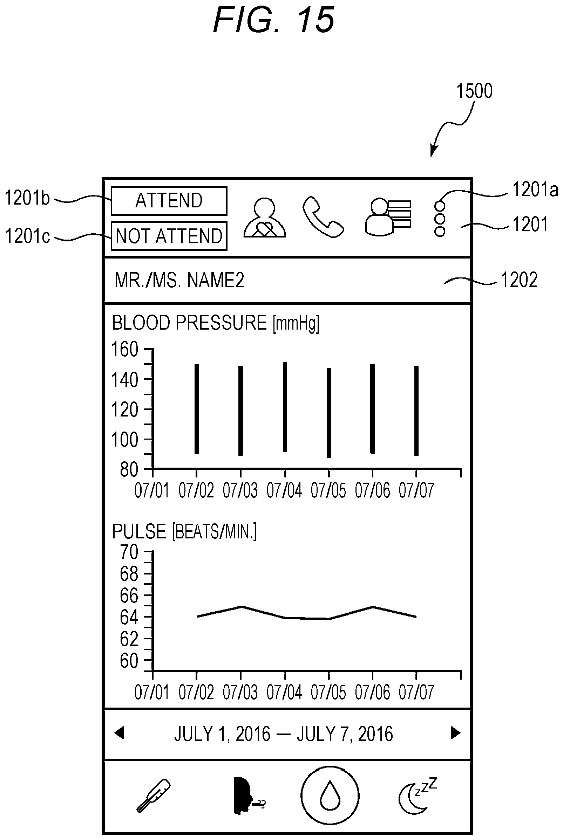

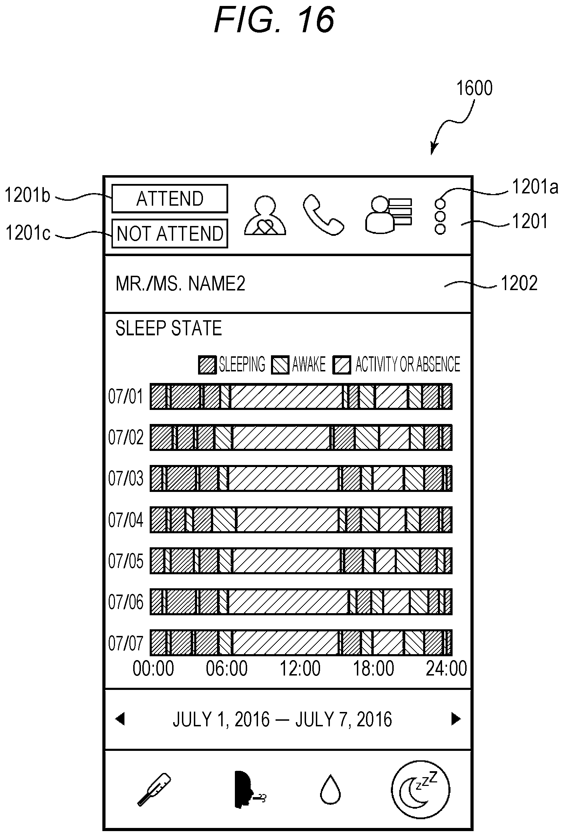

As a result, on the monitoring information screen 1200 of Fig. 12: when input operation (tapping) is performed on the body temperature display area 1203a, a body temperature screen 1300 illustrated in Fig. 13 is displayed on the TA display unit 36; when input operation (tapping) is performed on the micro body movement number display area 1203b, a micro body movement number screen 1400 illustrated in Fig. 14 is displayed on the TA display unit 36; when input operation (tapping) is performed on the blood pressure display area 1203c, a blood pressure screen 1500 illustrated in Fig. 15 is displayed on the TA display unit 36; and when input operation (tapping) is performed on the sleep state display area 1203d, a sleep state screen 1600 illustrated in Fig. 16 is displayed on the TA display unit 36.

In a lower end of each of the screens 1300, 1400, 1500, and 1600, a body temperature button 1211, a micro body movement number button 1212, a blood pressure button 1213, and a sleep state button 1214 are displayed in the order from the left. Each of the buttons 1211 to 1214 includes link destination information of corresponding vital sign information. When input operation (tapping) is performed on each of the buttons 1211 to 1214, the operation content is transmitted from the mobile terminal device TA-1 to the management server device SV. When the operation content is received, the SV monitoring processor 222 of the management server device SV transmits a display screen including the corresponding individual vital sign information to the mobile terminal device TA-1. As a result, when the input operation (tapping) is performed on each of the buttons 1211 to 1214, the corresponding screen 1300, 1400, 1500, or 1600 is displayed on the TA display unit 36.

A user of the mobile terminal device TA-1 (the nurse NA in Fig. 8) confirms, for example, the monitoring information screen 1200 of Fig. 12 or the micro body movement number screen 1400 of Fig. 14, and then determines whether to attend to the monitoring object person Ob-2 (name NAME2). On the monitoring information screen 1200 of Fig. 12 or the micro body movement number screen 1400 of Fig. 14, input operation (tapping) is performed on the "ATTEND" button 1201b, and then, input operation (tapping) is performed on one of the body temperature button 1211, the blood pressure button 1213, and the sleep state button 1214 (step S4010 in Fig. 8).

When the operation content is received, the SV monitoring processor 222 of the management server device SV transmits the display screen including the corresponding vital sign information to the mobile terminal device TA-1 (step S2040 in Fig. 8, an exemplary information transmitting step), and the corresponding screen out of the screens 1300, 1500, and 1600 is displayed on the TA display unit 36.

In step S4020, when the user of the mobile terminal device TA-1 (the nurse NA in Fig. 8) inputs an attendance policy by using the TA input unit 35, the input attendance policy is transmitted from the mobile terminal device TA-1 to the management server device SV. Examples of the "attendance policy" include direct attendance, conversation with the monitoring object person Ob-2 through a telephone, and the like in the present embodiment. In the present embodiment, examples of the "direct attendance" include a home visit by a doctor, home visit nursing by a nurse, home visit care by a care worker, and the like.

In Fig. 8, for example, the nurse NA goes to the house HS-2 of the monitoring object person Ob-2 as the direct attendance and provides home visit nursing. When the fact of providing the direct attendance is transmitted from the mobile terminal device TA-1 to the management server device SV in step S4020, the SV monitoring processor 222 of the management server device SV extracts an address of the monitoring object person Ob-2 from the SV sensor information table ST-SV (Fig. 6), and may transmit the extracted address of the monitoring object person Ob-2 to the mobile terminal device TA-1 via the SV communication IF unit 21. Alternatively, the information same as the SV sensor information table ST-SV (Fig. 6) may be preliminarily saved in the TA storage unit 33 of the mobile terminal device TA-1.

In step S2050, the management server device SV transmits, to the fixed terminal device SP in the call center CS, the person who attends to the monitoring object person Ob-2 and an attending situation. In step S3010, the fixed terminal device SP displays, on the display unit DP, the person who attends to the monitoring object person Ob-2 (the nurse NA in Fig. 8) and the attending situation (direct attendance in Fig. 8).

In step S4030, the user of the mobile terminal device TA-1 (the nurse NA in Fig. 8) goes to the house HS-2 of the monitoring object person Ob-2 and provides the home visit nursing.

In step S4040, the mobile terminal device TA-1 transmits a recording format request to the management server device SV in order to input a record of the home visit nursing. Subsequently, in step S2060, the management server device SV transmits the requested recording format to the mobile terminal device TA-1 as a reply. Specifically, transmission of the recording format request and reply transmission of the requested recording format are performed as follows.

For example, when the input operation on the submenu button 1201a of the menu bar area 1201 is received by the TA input unit 35 on the monitoring information screen 1200 of Fig. 12 or the micro body movement number screen 1400 of Fig. 14, the TA nursing care recording processor 325 of the mobile terminal device TA-1 displays, on the TA display unit 36, the submenu screen 61 illustrated in Fig. 17, for example. The submenu button 1201a illustrated in each of Figs. 12 to 16 is a button to input, to the mobile terminal device TA-1, an instruction to display the submenu screen 61.

The submenu screen 61 is a screen to display selectable submenus and select a submenu. As illustrated in Fig. 17, for example, the submenu screen 61 includes an "INFORMATION" button 611, a "STAFF MESSAGE BOARD" button 612, an "INPUT OF PROVIDED CARE" button 613, and a "LOG OUT" button 614.

The "INFORMATION" button 611 is a button to input, to the mobile terminal device TA-1, an instruction to display predetermined information such as a login name (the nurse NA in the present embodiment), a terminal ID, and the like. The "STAFF MESSAGE BOARD" button 612 is a button to input, to the mobile terminal device TA-1, an instruction to display a message registered on an electronic message board recorded in the management server device SV by the monitoring person (user) from the mobile terminal device TA-1.

The "INPUT OF PROVIDED CARE" button 613 is a button to input, to the mobile terminal device TA-1, an instruction to record a nursing care record in the management server device SV by the monitoring person from the mobile terminal device TA-1. The "LOG OUT" button 614 is a button to input, to the mobile terminal device TA-1, an instruction to log out.

When input operation on the "INPUT OF PROVIDED CARE" button 613 is received by the TA input unit 35 in a state in which the submenu screen 61 illustrated in Fig. 17 is displayed on the TA display unit 36, the TA nursing care recording processor 325 of the mobile terminal device TA-1 displays the format selection screen 63 illustrated in Fig. 18 on the TA display unit 36, for example.

The format selection screen 63 is a screen to display selectable recording formats prepared in advance in accordance with kinds of nursing care, and select a recording format. For example, as illustrated in Fig. 18, the format selection screen 63 includes the menu bar area 1201, the user name display area 1202, a format display area 632 (632-1 to 632-4) in which one or a plurality of recording formats is displayed with a name or names thereof in a list form.

In the example illustrated in Fig. 18, the "NAME2" that is the name of the monitoring object person Ob-2 is displayed in the user name display area 1202. Note that in a case where the format display area 632 is wider than the display area of the TA display unit 36 and all of recording format names cannot be displayed in the display area of the TA display unit 36, an area portion of the format display area 632 to be displayed on the display area of the TA display unit 36 can be changed by, for example, flick input so that a recording format name not displayed on the display area of the TA display unit 36 can be displayed.

Each of the areas 632-1 to 632-4 to display the recording format names is a format selection button to input, to the mobile terminal device TA-1, a recording format selected by the monitoring person. In the example illustrated in Fig. 18, the format display area 632 includes: a "MEALS" area 632-1 displaying "MEALS" as a recording format name for the meal care; a "FLUID" area 632-2 displaying "FLUID" as a recording format name for the rehydration care; an "EXCRETION" area 632-3 displaying "excretion" as a recording format name for the excretion care; a "VITAL" area 632-4 displaying "VITAL" as a recording format name for the vital check.

The "MEALS" area 632-1 is a button to input a selection of the recording format for the meal care to the mobile terminal device TA-1, the "FLUID" area 632-2 is a button to input a selection of the recording format for the rehydration care to the mobile terminal device TA-1, the "EXCRETION" area 632-3 is a button to input a selection of the recording format for the excretion care to the mobile terminal device TA-1, and the "VITAL" area 632-4 is a button to input a selection of the recording format for the vital check to the mobile terminal device TA-1.

Additionally, as illustrated in Fig. 18, the content of the nursing care record having been provided until the present time may also be displayed in each of the areas 632-1 to 632-4 to display the recording format names. For example, respective content of nursing care records of today, the previous day, and the previous time may be displayed. To display the respective content of nursing care records in the area 632, the TA nursing care recording processor 325 may transmit, to the management server device SV, a communication signal containing an order to request the own terminal ID, an object person name (monitoring object person name) to whom the direct attendance has been provided, and respective content of nursing care records of today, the previous day, and the previous time in order to request the content of each of the nursing care record.

When the communication signal to request the nursing care records is received, the management server device SV may retrieve, from the nursing care record information storage unit 235, the respective content of nursing care records of today, the previous day, and the previous time corresponding to the object person name (monitoring object person name) contained in the received communication signal to request the nursing care records, and the management server device SV may transmit, as a reply to the mobile terminal device TA that is a transmission source of the communication signal to request the nursing care records, a communication signal in order to transmit, as a reply, the nursing care records containing the retrieved respective content of nursing care records of today, the previous day, and the previous time as well as the terminal ID, and the name of the object person name (monitoring object person name) contained in the received communication signal to request the nursing care records. The TA nursing care recording processor 325 receives the communication signal to transmit the nursing care records as the reply from the management server device SV, and may display the respective content of nursing care records on the format display area 632 by retrieving the contained data.

While the format selection screen 63 is displayed (Fig. 18), the TA nursing care recording processor 325 of the mobile terminal device TA receives a selection of a recording format name by using the TA input unit 35 through input operation on the format display area 632 that is the area displaying the recording format names. Then, the TA nursing care recording processor 325 transmits, to the management server device SV via the TA communication IF unit 31, a communication signal containing an order to request the own terminal ID and a recording format of the selected recording format name (step S4040 in Fig. 8).

When the communication signal is received, the format distribution processor 223 of the management server device SV retrieves, from the format information storage unit 234, an electronic file of the recording format corresponding to the recording format name contained in the received communication signal, and transmits, as a reply, a communication signal containing the retrieved electronic file of the recording format to the mobile terminal device TA-1 of the transmission source terminal ID via the SV communication IF unit 21 (step S2060 in Fig. 8).

When the reply from the management server device SV is received while the format selection screen 63 (Fig. 18) is displayed, the TA nursing care recording processor 325 of the mobile terminal device TA-1 retrieves the electronic file of the recording format contained in the received communication signal, and displays the recording format screen 64 on the TA display unit 36 by using the retrieved electronic file of the recording format.

For example, input operation is performed on the format display area 632-4 that is the area displaying the "VITAL" on the format selection screen 63. Then, the TA nursing care recording processor 325 of the mobile terminal device TA-1 transmits, to the management server device SV via the TA communication IF unit 31, a communication signal containing an order to request a recording format for vital check. Then, the mobile terminal device TA-1 receives the communication signal containing an electronic file of the recording format for the vital check as the reply, and displays the recording format screen 64 for the vital check illustrated in Fig. 19 on the TA display unit 36, for example. In the present embodiment, since attendance is performed relative to the increase in the respiration rate of the monitoring object person Ob-2, body temperature measurement is performed by the nurse NA.

The recording format screen 64 for the vital check in Fig. 19 is the screen that displays the recording format for the vital check and is a screen to input content of the performed vital check as a nursing care record. For example, as illustrated in Fig. 19, the recording format screen 64 for the vital check includes: the menu bar area 1201, the user name display area 1202; a nursing care record input area 641 displaying input items to be input as the content of nursing care, and used to input the content of nursing care in accordance with the input items as a nursing care record; a sharing checkbox (□) 642 to input a setting for whether to enable the nursing care record to be referred from the fixed terminal device SP and other mobile terminal devices TA; and a "TRANSMIT" button 643.

In the recording format for the vital check, for example, a body temperature, a memo, and the care providing time are set as the input items in the present embodiment. Additionally, to input these items, the nursing care record input area 641 includes: an input area for a body temperature 641-1 to input a body temperature; an input area for free remarks 641-2 to input memos; and an input area for the care providing time 641-3 to input the time when nursing care is provided.

When the sharing check box 642 receives input operation of a tick, "✔" is displayed in the sharing check box 642, and an instruction is input to provide a setting in which the content that has been input to the recording format screen 64 for the vital check can be referred from the fixed terminal device SP and the other mobile terminal devices TA. On the other hand, in a case of not receiving the input operation of the tick, the inside of the sharing check box 642 is displayed blank, and an instruction is input to provide a setting in which the content that has been input to the recording format screen 64 for the vital check cannot be referred from the fixed terminal device SP and the other mobile terminal devices TA. The "TRANSMIT" button 643 is a button to input an instruction to transmit, to the management server device SV, the content that has been input to the recording format screen 64 for the vital check.

Returning to Fig. 8, in step S4050, when inputting an attendance result is completed and input operation is performed on the "TRANSMIT" button 643 by the user (the nurse NA in Fig. 8), the TA nursing care recording processor 325 of the mobile terminal device TA-1 transmits, to the management server device SV via the TA communication IF unit 31, a communication signal containing the own device terminal ID, the content of the attendance result input by using the recording format screen 64 for vital check (in the above example, the body temperature, the memo, the care providing time, and the allowance/non-allowance for sharing).

In step S2070, the SV monitoring processor 222 of the management server device SV having received the communication signal saves, in the nursing care record information storage unit 235, the content of the attendance result contained in the communication signal. In step S2080, the SV monitoring processor 222 of the management server device SV notifies the fixed terminal device SP of a fact that the attendance to the monitoring object person Ob-2 is completed. In step S3020, with receipt of this notification, the fixed terminal device SP displays, on the display unit DP, the fact that the attendance is completed.

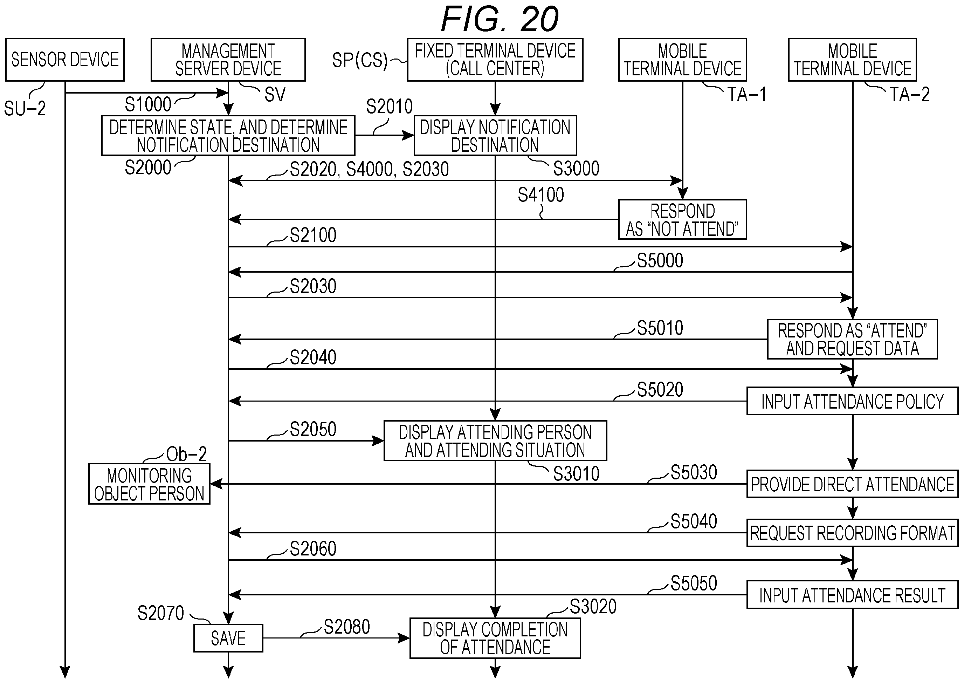

Fig. 20 is a sequence diagram schematically illustrating exemplary operation in the assistance system MS different from the operation in Fig. 8. In Fig. 20, steps S1000, S2000, S2010, S3000, S2020, S4000, and S2030 are the same as steps S1000, S2000, S2010, S3000, S2020, S4000, and S2030 in Fig. 8.

Similar to Fig. 8, in step S2030, the SV monitoring processor 222 of the management server device SV transmits the latest monitoring information of the monitoring object person Ob-2 to the mobile terminal device TA-1. As illustrated in Fig. 12, the TA monitoring processor 322 of the mobile terminal device TA-1 displays, on the TA display unit 36, the monitoring information screen 1200 representing the monitoring information of the monitoring object person Ob-2 transmitted from the management server device SV.

In a state in which the monitoring information screen 1200 of Fig. 12 is displayed on the TA display unit 36, input operation (tapping) is performed on the "NOT ATTEND" button 1201c by the nurse NA in step S4100 of Fig. 20. A reason may be that the nurse NA stays far from the house HS-2 of the monitoring object person Ob-2 and cannot attend immediately, or the nurse NA is currently providing care for a different monitoring object person Ob and is not available, for example.

With receipt of this operation content, the SV monitoring processor 222 of the management server device SV refers to the notification destination field 2322 of the notification destination information table AT (Fig. 5A) in the terminal device information storage unit 232, and acquires "TA-2" next to "TA-1" in step S2100, and further refers to the terminal information table DT (Fig. 5B) in the terminal device information storage unit 232 to acquire a communication address of the mobile terminal device TA-2. The SV monitoring processor 222 notifies the acquired communication address of the mobile terminal device TA-2 of the abnormality of the monitoring object person Ob-2.

Subsequent steps S2030 to S2080 in the management server device SV are the same as steps S2030 to S2080 of Fig. 8 except that: a communication partner is the mobile terminal device TA-2 instead of the mobile terminal device TA-1; and an attending person for the monitoring object person Ob-2 to be transmitted to the fixed terminal device SP of the call center CS in step S2050 is not the nurse NA but the nurse NB.

Additionally, in subsequent steps S3010 and S3020 in the fixed terminal device SP are same as steps S3010 and S3020 of Fig. 8 except that the attending person displayed on the display unit DB is not the nurse NA but the nurse NB. Furthermore, subsequent steps S5000 to S5050 in the mobile terminal device TA-2 are the same as steps S4000 to S4050 of Fig. 8 except that an operation subject is different from the mobile terminal device TA-1.

Note that, in the present embodiment, the notification destination mobile terminal device is determined by ranking the notification destinations from the first to the third as illustrated in the notification destination information table AT in Fig. 5A. Therefore, in Fig. 20, when input operation is performed on the "NOT ATTEND" button 1201c also in the mobile terminal device TA-2, the SV monitoring processor 222 of the management server device SV notifies the third mobile terminal device TA-8 of the abnormality of the monitoring object person Ob-2.

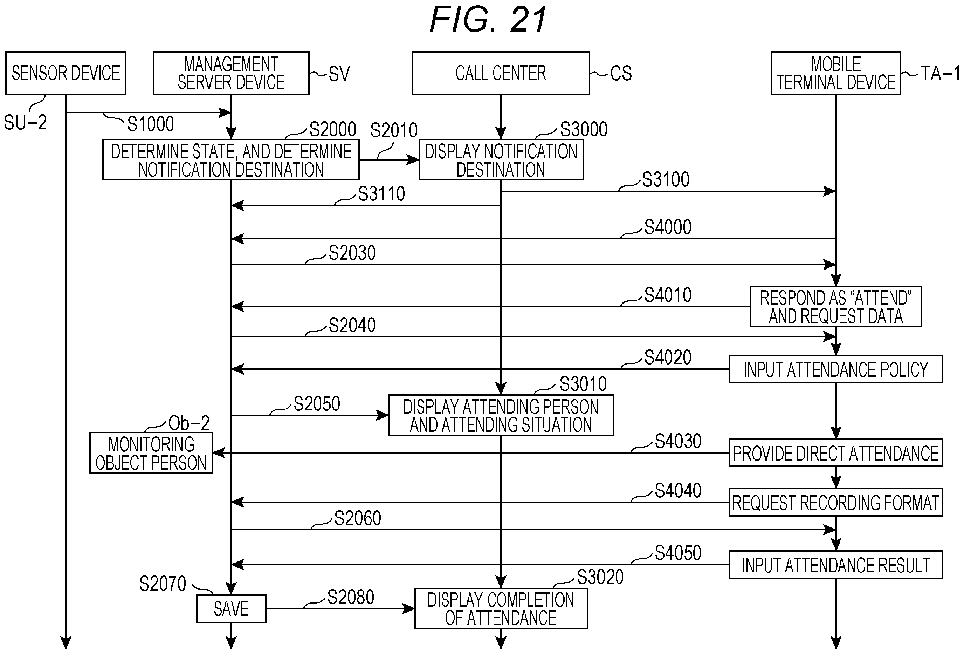

Fig. 21 is a sequence diagram schematically illustrating exemplary operation different from operation in the assistance systems MS of Figs. 8 and 20. In Fig. 21, steps S1000, S2000, S2010, S3000 are the same as steps S1000, S2000, S2010, S3000 of Fig. 8.

In step S3000, similar to Fig. 8, the notification destination transmitted from the management server device SV is displayed on the display unit DP of the fixed terminal device SP. In this state, in Fig. 21, a staff member of the call center CS operates the fixed terminal device SP and notifies, of the abnormal state of the monitoring object person Ob-2, the notification destination mobile terminal device TA-1 displayed on the display unit DP in step S3100. With receipt of this notification, the login processor 326 of the mobile terminal device TA-1 displays, on the TA display unit 36 of the mobile terminal device TA-1, the notification screen 900 stored in the screen information storage unit 333, as illustrated in Fig. 9. However, the information transmitted from the fixed terminal device SP is used for the "NAME2" in the message column 901.

In step S3100, the staff member of the call center CS may operate the fixed terminal device SP and refer to the terminal information table DT (Fig. 5B) of the terminal device information storage unit 232 of the management server device SV to acquire the communication address of the mobile terminal device TA-1. Alternatively, the fixed terminal device SP may preliminarily store information same as the information of the terminal information table DT (Fig. 5B) in the storage unit.

In step S3110, the staff member of the call center CS operates the fixed terminal device SP and notifies the management server device SV of a fact that the mobile terminal device TA-1 has been notified of the abnormality of the monitoring object person Ob-2. Operation after step S4000 is the same as that of Fig. 8.

As described above, in the assistance system MS and the assistance control method implemented in the assistance system MS in the present embodiment, the SV monitoring processor 222 of the management server device SV determines whether each monitoring object person Ob is in an abnormal state based on detection data transmitted from the sensor device SU. In the present embodiment, when it is determined that the monitoring object person Ob is in the abnormal state, the SV monitoring processor 222 determines, from among the plurality of mobile terminal devices TA, a notification destination mobile terminal device TA to be notified of the abnormality of the monitoring object person Ob by ranking the mobile terminal devices from the first to the third in accordance with the abnormal state. Therefore, according to the present embodiment, an appropriate monitoring person (user) can be notified of the abnormality of the monitoring object person Ob when the monitoring object person Ob is in the abnormal state.

The monitoring information screen 1200 (Fig. 12) is displayed on the TA display unit 36 of the notification destination mobile terminal device TA-1 that has been notified of the abnormality of the monitoring object person Ob-2, and when input operation (tapping) is performed on each of the personalized display areas 1203a to 1203d on the monitoring information screen 1200 of Fig. 12, each of the personalized vital sign information screens 1300 to 1600 (Figs. 13 to 16) is displayed on the TA display unit 36. Therefore, according to the present embodiment, the user (the nurse NA in the present embodiment) of the mobile terminal device TA-1 that has been notified of the abnormality of the monitoring object person Ob can confirm the state of the monitoring object person Ob.

Additionally, in the present embodiment, when input operation (tapping) is performed on the "NOT ATTEND" button 1201c in the notification destination mobile terminal device TA-1 that has been notified of the abnormality of the monitoring object person Ob-2, the second mobile terminal device TA-2 is notified of the abnormality of the monitoring object person Ob-2. Therefore, according to the present embodiment, it is possible to more reliably notify the appropriate notification destination of the abnormality of the monitoring object person Ob-2.

Thus, according to the present embodiment, the user using the notification destination terminal device that has been notified of the abnormality of the monitoring object person can confirm the monitoring object person information transmitted from the central processing device.

### (Modified Embodiment)

(1) In an above-described embodiment, a nurse NA using a notification destination mobile terminal device TA-1 that has been notified of abnormality of a monitoring object person Ob-2 goes to a house HS-2 of the monitoring object person Ob-2 and provides direct attendance, but attendance to the monitoring object person Ob-2 is not limited to the direct attendance. For example, the nurse NA using the mobile terminal device TA-1 may also perform, as attendance to the monitoring object person Ob-2, conversation with the monitoring object person Ob-2 through voice communication between the mobile terminal device TA-1 and a sensor device SU-2.
(2) In the above-described embodiment of Fig. 21, a notification destination transmitted from a management server device SV is displayed on a display unit DP of a fixed terminal device SP in step S3000 in a manner similar to Fig. 8. Then, in step S3100 of Fig. 21, a staff member of a call center CS operates the fixed terminal device SP to notify, of an abnormality of the monitoring object person Ob-2, the notification destination mobile terminal device TA-1 displayed on the display unit DP.
   Alternatively, the staff member of the call center CS may confirm, based on detection data of a sensor device SU, a body temperature, the number of micro body movements, a blood pressure, a pulse, a sleep state of a monitoring object person Ob determined by an SV monitoring processor 222 of the management server device SV, and may determine, by himself/herself, a mobile terminal device TA used by a monitoring person appropriate for the state of the monitoring object person Ob, and then notify the determined mobile terminal device TA of an abnormality of the monitoring object person Ob by using the fixed terminal device SP.
(3) In the above-described embodiment, link destination information of corresponding vital sign information is included in each of display areas 1203a, 1203b, 1203c, and 1203d included in a vital sign information display area 1203 of Fig. 12. Consequently, when input operation (tapping) is performed on each of the display areas 1203a, 1203b, 1203c, and 1203d on a monitoring information screen 1200 illustrated in Fig. 12, operation content thereof is transmitted from the mobile terminal device TA-1 to the management server device SV, and the SV monitoring processor 222 of the management server device SV transmits each of the corresponding personalized vital sign information display screens 1300, 1400, 1500, and 1600 (Figs. 13 to 16) to the mobile terminal device TA-1.

In this case, a valid term may be provided in the link destination information of the corresponding vital sign information included in each of the display areas 1203a, 1203b, 1203c, and 1203d. In other words, the SV monitoring processor 222 of the management server device SV may be set so as not transmit each of the corresponding personalized vital sign information display screens 1300, 1400, 1500, and 1600 (Figs. 13 to 16) to the mobile terminal device TA-1 after elapse of a predetermined time from a time point transmission of the monitoring information screen 1200 of Fig. 12 from the management server device SV to the mobile terminal device TA-1, even though input operation (tapping) is performed on each of the display areas 1203a, 1203b, 1203c, and 1203d.

Consequently, a time width during which the personalized vital sign information display screens 1300, 1400, 1500, and 1600 (Figs. 13 to 16) are transmitted from the management server device SV to the mobile terminal device TA-1 is limited. As a result, a possibility of leaking the personalized vital sign information display screens 1300, 1400, 1500, and 1600 (Figs. 13 to 16) to the outside can be reduced.

Alternatively, when input operation (tapping) is performed on a "NOT ATTEND" button 1201c in a state in which the monitoring information screen 1200 of Fig. 12 is displayed on the mobile terminal device TA-1, the SV monitoring processor 222 of the management server device SV may be set so as not to transmit each of the corresponding personalized vital sign information display screens 1300, 1400, 1500, and 1600 (Figs. 13 to 16) to the mobile terminal device TA-1 after the operation content is transmitted to the management server device SV, even though input operation (tapping) is performed on each of the display areas 1203a, 1203b, 1203c, and 1203d.

Or else, when input operation (tapping) is performed on the "NOT ATTEND" button 1201c in the state in which the monitoring information screen 1200 of Fig. 12 is displayed on the mobile terminal device TA-1, a TA monitoring processor 322 may display, on a TA display unit 36, a submenu screen 61 (Fig. 17) instead of the monitoring information screen 1200.

Even in these cases, since the monitoring person using the mobile terminal device TA-1 (the nurse NA in the above embodiment) does not attend to the monitoring object person Ob-2, there is no problem. Furthermore, this can reduce the possibility of leaking the personalized vital sign information display screens 1300, 1400, 1500, and 1600 (Figs. 13 to 16) to the outside.

(4) In the above-described embodiment, the monitoring object person Ob is a solitary person who lives alone, and the fixed terminal device SP of an assistance system MS is arranged in the call center CS. However, as described in the above embodiment, the assistance system MS is preferably arranged in a building such as a hospital, an elderly welfare facility, or a dwelling in accordance with a type of each monitoring object person Ob.

For example, the monitoring object person Ob may be a resident who lives in a care home or a patient hospitalized in a hospital. In this case, the assistance system MS is arranged in the care home or the hospital, and the sensor device SU is arranged in a room of the resident of the care home or a hospital room of the patient in the hospital, and the fixed terminal device SP is arranged in the care home or a nurse station of the hospital.

In this modified embodiment, a notification destination terminal device to be notified of abnormality of a monitoring object person Ob by the management server device SV is not limited to the mobile terminal device TA and may include the fixed terminal device SP. In a case where the fixed terminal device SP is notified of the abnormality of the monitoring object person Ob by the management server device SV, the management server device SV may omit display of a notification screen 900 (Fig. 9) and may display the monitoring information screen 1200 (Fig. 12) on the display unit DP of the fixed terminal device SP. An on-call person in the nurse station may perform input operation (such as clicking with a mouse) on a "ATTEND" button 1201b on the monitoring information screen 1200 (Fig. 12) displayed on the display unit DP, for example.

While the present specification discloses various modes of the technologies as described above, the main technologies thereof are summarized below.

A mode of the present invention is an assistance control method for an assistance system that assists monitoring a monitoring object person and includes:
a sensor device which is provided in a manner corresponding to the monitoring object person who is a monitoring target, and detects monitoring object person information that is information associated with the monitoring object person;
a central processing device which is communicably connected to the sensor device, receives, from the sensor device, the monitoring object person information detected by the sensor device, and determines an abnormal state of the monitoring object person based on the monitoring object person information; and
a plurality of terminal devices communicably connected to the central processing device, and
the method includes:
   an acquisition step of acquiring, from among the plurality of terminal devices, a notification destination terminal device which is a terminal device of a notification destination to be notified of abnormality of the monitoring object person, and is determined in accordance with an abnormal state of the monitoring object person determined by the central processing device;
   an abnormality notification step of notifying, of the abnormality of the monitoring object person, the notification destination terminal device acquired in the acquisition step; and
   an information transmission step of transmitting the monitoring object person information to the notification destination terminal device from the central processing device in response to a transmission request for the monitoring object person information from the notification destination terminal device that has been notified of abnormality of the monitoring object person in the abnormality notification step.

In the present mode, the notification destination terminal device which is the terminal device of the notification destination to be notified of the abnormality of the monitoring object person and is determined in accordance with the abnormal state of the monitoring object person determined by the central processing device is acquired from among the plurality of terminal devices. The acquired notification destination terminal device is notified of the abnormality of the monitoring object person. The monitoring object person information is transmitted from the central processing device to the notification destination terminal device in response to the transmission request for the monitoring object person information from the notification destination terminal device that has been notified of the abnormality of the monitoring object person.

Therefore, according to the present mode, the user using the notification destination terminal device that has been notified of the abnormality of the monitoring object person can confirm the monitoring object person information. For this reason, the user can determine whether to attend to the monitoring object person after confirming the monitoring object person information, for example.

In the above-described assistance control method, in a case where the abnormal state is a state in which the monitoring object person has collapsed or fallen from a bed, a terminal device used by a care worker may be determined as the notification destination terminal device, and in a case where the abnormal state is a state in which a respiration rate of the monitoring object person is increased by a predetermined percentage or more from a maximum value in a variation range of a normal state, a terminal device used by a nurse may be determined as the notification destination terminal device. In a case where the abnormal state is a state in which a body temperature of the monitoring object person is a predetermined temperature or higher than an upper limit value in variation of the normal state, a terminal device used by a doctor may be determined as the notification destination terminal device, and in a case where the abnormal state is a state in which a sleep state of the monitoring object person Ob is reversed between daytime and night time, a terminal device used by a family member of the monitoring object person may be determined as the notification destination terminal device.

In the above-described mode, for example, it may be possible to further include: a result transmission step of transmitting, from the notification destination terminal device to the central processing device, a result of attendance provided for the monitoring object person; and a saving step of saving, in the central processing device, the result of attendance transmitted from the notification destination terminal device to the central processing device in the result transmission step.

In the present mode, the result of attendance provided for the monitoring object person is transmitted from the notification destination terminal device to the central processing device. The attendance result transmitted from the notification destination terminal device to the central processing device is saved in the central processing device. Therefore, according to the present mode, a result of having attended to the abnormal state of the monitoring object person can be confirmed later.

In the above-described mode, for example, the information transmission step may further include transmitting the monitoring object person information to the notification destination terminal device from the central processing device in accordance with the transmission request only during a period in which a predetermined time elapses from a time point when the notification destination terminal device is notified of the abnormality of the monitoring object person in the abnormality notification step.

In the present mode, the monitoring object person information is transmitted from the central processing device to the notification destination terminal device in accordance with the transmission request only during the period in which the predetermined time elapses from the time point when the notification destination terminal device is notified of the abnormality of the monitoring object person. Therefore, according to the present mode, the period during which the monitoring object person information is transmitted from the central processing device to the notification destination terminal device is limited. As a result, the possibility of leaking the monitoring object person information to the outside can be reduced.

In the present mode, it may be possible to further include, for example: an attendance unavailable signal transmission step of transmitting an attendance unavailable signal to the central processing device from the notification destination terminal device when input operation indicating that no attendance can be provided for the abnormality of the monitoring object person is performed on the notification destination terminal device; and a new abnormality notification step of notifying, of the abnormality of the monitoring object person, another terminal device, which is different from the notification destination terminal device out of the plurality of terminal devices, when the attendance unavailable signal is transmitted to the central processing device from the notification destination terminal device in the attendance unavailable signal transmission step.

In the present mode, when the input operation indicating that no attendance can be provided for the abnormality of the monitoring object person is performed on the notification destination terminal device, the attendance unavailable signal is transmitted from the notification destination terminal device to the central processing device. When the attendance unavailable signal is transmitted from the notification destination terminal device to the central processing device, another terminal device which is different from the notification destination terminal device out of the plurality of terminal devices is notified of the abnormality of the monitoring object person. Therefore, according to the present mode, even in a case where the user of the notification destination terminal device cannot attend to the abnormality of the monitoring object person, a user of another terminal device can attend to the abnormality of the monitoring object person by notifying another terminal device of the abnormality of the monitoring object person.

In the above mode, for example, when the attendance unavailable signal is transmitted from the notification destination terminal device to the central processing device in the attendance unavailable signal transmission step, the information transmission step may stop transmission of the monitoring object person information from the central processing device to the notification destination terminal device.

In the present mode, when the attendance unavailable signal is transmitted from the notification destination terminal device to the central processing device, the transmission of the monitoring object person information from the central processing device to the notification destination terminal device is stopped. Therefore, according to the present mode, the monitoring object person information is not transmitted to the notification destination terminal device used by the user who cannot attend to the abnormality of the monitoring object person. As a result, the possibility of leaking the monitoring object person information to the outside can be reduced.

Another mode of the present invention is an assistance system that assists monitoring a monitoring object person who is a monitoring target and includes:
a sensor device which is provided in a manner corresponding to the monitoring object person and detects monitoring object person information that is information associated with the monitoring object person;
a central processing device communicably connected to the sensor device and adapted to receive, from the sensor device, the monitoring object person information detected by the sensor device; and
a plurality of terminal devices communicably connected to the central processing device, in which
the central processing device includes:
   a first monitoring processor adapted to determine an abnormal state of the monitoring object person based on the monitoring object person information, and determine, from among the plurality of terminal devices, a notification destination terminal device which is a notification destination terminal device to be notified of abnormality of the monitoring object person in accordance with the determined abnormal state of the monitoring object person; and
   a first communicator that notifies, of the abnormality of the monitoring object person, the notification destination terminal device determined by the first monitoring processor,
   the notification destination terminal device includes
   a second communicator adapted to transmit a transmission request for the monitoring object person information to the central processing device when the notification of the abnormality of the monitoring object person is provided from the central processing device, and
   the first communicator is adapted to acquire the monitoring object person information from the first monitoring processor and transmit the monitoring object person information to the notification destination terminal device when the transmission request for the monitoring object person information is transmitted from the notification destination terminal device.

In the present mode, the monitoring object person information, which is the information associated with the monitoring object person, is detected by the sensor device provided in the manner corresponding to the monitoring object person that is the monitoring target. The abnormal state of the monitoring object person is determined based on the monitoring object person information. The notification destination terminal device, which is the terminal device of the notification destination to be notified of the abnormality of the monitoring object person, is determined from among the plurality of terminal devices in accordance with the determined abnormal state of the monitoring object person. The determined notification destination terminal device is notified of the abnormality of the monitoring object person. When the notification of the abnormality of the monitoring object person is received from the central processing device, the transmission request for the monitoring object person information is transmitted to the central processing device. When the transmission request for the monitoring object person information is transmitted from the notification destination terminal device, the monitoring object person information is acquired from the first monitoring processor and transmitted to the notification destination terminal device.

Therefore, according to the present mode, a user using the notification destination terminal device that has been notified of the abnormality of the monitoring object person can confirm the monitoring object person information transmitted from the central processing device. For this reason, the user can determine whether to attend to the monitoring object person after confirming the monitoring object person information, for example.

The present invention has been appropriately and fully described above through the embodiments with reference to the drawings to depict the present invention, but it should be recognized that a modification and/or an improvement is/are easily conceivable by those skilled in the art. Therefore, such a modified or improved embodiment achieved by those skilled in the art is understood as being included within the scope of the rights of the claims unless otherwise the modified or improved embodiment is in a level departing from the scope of the rights of the claims recited in the scope of the claims.

The embodiments of the present invention have been illustrated and described in detail, but note that the embodiments are simply illustrated examples and practical examples, and not intended to be limitative. The scope of the present invention should be construed by the wordings of the appended claims.

The entire disclosure of Japanese Patent Application No. 2016-245203 filed on December 19, 2016 is hereby incorporated by reference in its entirety.

## Claims

1. An assistance control method for an assistance system that assists monitoring a monitoring object person and includes:
a sensor device which is provided in a manner corresponding to the monitoring object person who is a monitoring target, and detects monitoring object person information that is information associated with the monitoring object person;
a central processing device which is communicably connected to the sensor device, receives, from the sensor device, the monitoring object person information detected by the sensor device, and determines an abnormal state of the monitoring object person based on the monitoring object person information; and
a plurality of terminal devices communicably connected to the central processing device,
the method comprising:
an acquisition step of acquiring, from among the plurality of terminal devices, a notification destination terminal device which is a terminal device of a notification destination to be notified of abnormality of the monitoring object person and is determined in accordance with the abnormal state of the monitoring object person determined by the central processing device;
an abnormality notification step of notifying, of the abnormality of the monitoring object person, the notification destination terminal device acquired in the acquisition step; and
an information transmission step of transmitting the monitoring object person information to the notification destination terminal device from the central processing device in response to a transmission request for the monitoring object person information from the notification destination terminal device that has been notified of abnormality of the monitoring object person in the abnormality notification step.

2. The assistance control method according to claim 1, further comprising:
a result transmission step of transmitting, from the notification destination terminal device to the central processing device, a result of attendance provided for the monitoring object person; and
a saving step of saving, in the central processing device, the result of attendance transmitted from the notification destination terminal device to the central processing device in the result transmission step.

3. The assistance control method according to claim 1 or 2, wherein
the information transmission step further includes transmitting the monitoring object person information to the notification destination terminal device from the central processing device in accordance with the transmission request only during a period in which a predetermined time elapses from a time point when the notification destination terminal device is notified of the abnormality of the monitoring object person in the abnormality notification step.

4. The assistance control method according to any one of claims 1 to 3 further comprising:
an attendance unavailable signal transmission step of transmitting an attendance unavailable signal to the central processing device from the notification destination terminal device when input operation indicating that no attendance can be provided for the abnormality of the monitoring object person is performed on the notification destination terminal device; and
a new abnormality notification step of notifying, of the abnormality of the monitoring object person, another terminal device, which is different from the notification destination terminal device out of the plurality of terminal devices, when the attendance unavailable signal is transmitted to the central processing device from the notification destination terminal device in the attendance unavailable signal transmission step.

5. The assistance control method according to claim 4, wherein
the information transmission step stops transmission of the monitoring object person information from the central processing device to the notification destination terminal device when the attendance unavailable signal is transmitted from the notification destination terminal device to the central processing device in the attendance unavailable signal transmission step.

6. An assistance system that assists monitoring a monitoring object person who is a monitoring target, comprising:
a sensor device which is provided in a manner corresponding to the monitoring object person and detects monitoring object person information that is information associated with the monitoring object person;
a central processing device communicably connected to the sensor device and configured to receive, from the sensor device, the monitoring object person information detected by the sensor device;
a plurality of terminal devices communicably connected to the central processing device, wherein
the central processing device includes:
a first monitoring processor configured to determine an abnormal state of the monitoring object person based on the monitoring object person information, and determine, from among the plurality of terminal devices, a notification destination terminal device which is a notification destination terminal device to be notified of abnormality of the monitoring object person in accordance with the determined abnormal state of the monitoring object person; and
a first communicator that notifies, of the abnormality of the monitoring object person, the notification destination terminal device determined by the first monitoring processor,
the notification destination terminal device includes
a second communicator configured to transmit a transmission request for the monitoring object person information to the central processing device when the notification of the abnormality of the monitoring object person is provided from the central processing device, and
the first communicator is configured to acquire the monitoring object person information from the first monitoring processor and transmit the monitoring object person information to the notification destination terminal device when the transmission request for the monitoring object person information is transmitted from the notification destination terminal device.
